# EUROPEAN PATENT SPECIFICATION

(11) **EP 3 492 096 B1**
(45) Date of publication and mention of the grant of the patent: **18.11.2026**
(21) Application number: 17834796.9
(22) Date of filing: 27.07.2017
(51) Int. Cl.: A61K 38/46, A61K 48/00, C12N 9/22, A61P 27/02, C12N 15/10, C12N 15/113

(54) **PHARMACEUTICAL COMPOSITION FOR TREATING OCULAR DISEASES, CONTAINING CAS9 PROTEIN AND GUIDE RNA**
PHARMAZEUTISCHE ZUSAMMENSETZUNG ZUR BEHANDLUNG VON AUGENERKRANKUNGEN MIT CAS9-PROTEINS UND GUIDE-RNA
COMPOSITION PHARMACEUTIQUE POUR LE TRAITEMENT DE MALADIES OCULAIRES, CONTENANT UNE PROTÉINE CAS9 ET UN ARN GUIDE

(30) Priority: 28.07.2016 US 201662367674 P
(43) Date of publication of application: 05.06.2019
(73) Proprietor: Institute for Basic Science, Daejeon 34047 (KR); Seoul National University R&DB Foundation, Seoul 08826 (KR); Seoul National University Hospital, Seoul 03080 (KR)
(72) Inventor: KIM, Jin-Soo, Seoul 08831 (KR); KIM, Jeong Hun, Seoul 03080 (KR); PARK, Sung Wook, Seoul 03080 (KR); KIM, Kyoung Mi, Seoul 03024 (KR)
(74) Representative: Reddie & Grose LLP
(86) International application number: PCT/KR2017/008122
(87) International publication number: WO 2018/021855

(56) References cited:
- JP-A- 2015 177 801
- KR-A- 20160 089 527
- US-A1- 2015 376 586
- GLENN YIU ET AL: "ARVO Meeting Abstract; Genomic Disruption of VEGF in Human Retinal Pigment Epithelial Cells using CRISPR-Cas9 Endonuclease", IOVS, 1 May 2016 (2016-05-01), XP055675266, Retrieved from the Internet <URL:https://iovs.arvojournals.org/article.aspx?articleid=2559853&resultClick=1> [retrieved on 20200310]
- ANNE LOUISE ASKOU ET AL: "Reduction of choroidal neovascularization in mice by adeno-associated virus-delivered anti-vascular endothelial growth factor short hairpin RNA", JOURNAL OF GENE MEDICINE, vol. 14, no. 11, 1 November 2012 (2012-11-01), US, pages 632 - 641, XP055289335, ISSN: 1099-498X, DOI: 10.1002/jgm.2678
- KYOUNGMI KIM ET AL: "Genome surgery using Cas9 ribonucleoproteins for the treatment of age-related macular degeneration", GENOME RESEARCH, vol. 27, no. 3, 16 February 2017 (2017-02-16), US, pages 419 - 426, XP055636224, ISSN: 1088-9051, DOI: 10.1101/gr.219089.116
- YIU, GLENN ET AL.: "Genomic Disruption of VEGF in Human Retinal Pigment Epithelial Cells Using CRISPR-Cas9 Endonuclease", ARVO 2016 ANNUAL MEETING ABSTRACTS, 1 May 2016 (2016-05-01), XP055576195, Retrieved from the Internet <URL:http://www.arvo.org/webs/am2016/sectionpdf/BI/Session__161.pdf>
- KOO, TAEYOUNG ET AL.: "Measuring and Reducing Off-target Activities of Programmable Nucleases Including CRISPR-Cas9", MOLECULES AND CELLS, vol. 38, no. 6, 19 May 2015 (2015-05-19), pages 475 - 481, XP055372009, DOI: doi:10.14348/molcells.2015.0103
- YIU, GLENN ET AL.: "Genomic Disruption of VEGF-A Expression in Human Retinal Pigment Epithelial Cells Using CRISPR-Cas9 Endonuclease", INVESTIGATIVE OPHTHALMOLOGY & VISUAL SCIENCE, vol. 57, no. 13, August 2016 (2016-08-01), pages 5490 - 5497, XP055576195, DOI: doi:10.1167/iovs.16-20296

## Description

### TECHNICAL FIELD

Provided are a pharmaceutical composition for use in treating or preventing an eye disease associated with VEGF-A overexpression, the composition comprising a ribonucleoprotein comprising a Cas9 protein and a VEGF-A gene-specific guide RNA containing a targeting sequence binding specifically to a target site of a *VEGF-A* gene; wherein the guide RNA is complementary to a nucleic acid sequence of SEQ ID NO: 293 or 294 in a VEGF-A gene, and wherein the eye disease associated with VEGF-A overexpression is macular degeneration or retinopathy, and a ribonucloprotein comprising a Cas9 protein and a VEGF-A gene-specific guide RNA comprising a targeting sequence specifically binding to a target site of a *VEGF-A* gene, wherein the guide RNA is complementary to a nucleic acid sequence of SEQ ID NO: 293 or 294 in a VEGF-A gene.

### BACKGROUND OF THE INVENTION

RNA-guided genome surgery or RNA-guided genome editing using CRISPR-Cas9 nuclease is expected to be helpful for the therapy of various genetic diseases, but little is known about the therapeutic effects of CRISPR-Cas9 nuclease on non-genetic diseases.

One example representative of non-genetic diseases is macular degeneration (e.g., aged-related macular degeneration (AMD)). AMD is a major cause of blindness in the elderly population in advanced countries. Choroidal neovascularization (CNV) is a main pathological feature in neovascular AMD, and is principally caused by angiogenic cytokines such as vascular endothelial growth factor A (VEGF A). So far, development has mostly been made of monoclonal antibodies or aptamers targeting *VEGF-A,* as therapeutic agents for AMD. However, such *anti-VEGF-A* are problematic in that they must be administered seven times or more a year as *VEGF-A* is continually expressed and secreted in retinal cells.

Therefore, there is a need for the development of a more fundamental and long-lasting therapeutic technique for eye diseases.

Glenn Yiu et al., ARVO Meeting Abstract; Genomic Disruption of VEGF in Human Retinal Pigment Epithelial Cells using CRISPR-Cas9 Endonuclease. IOVS, 1 May 2016, describes a lentiviral vector encoding S. pyogenes Cas9 endonuclease and guide RNAs targeting VEGF-A, used to transfect ARPE-19 cells, resulting in indel formation in the VEGF-A gene.

### [Reference]

Korean Patent No. 10-2015-0101446 A (issued on September 03, 2015)

### DETAILED DESCRIPTION OF THE INVENTION

### [Technical Problem]

The present specification proposes a technique that allows for the long-lasting or permanent therapy of eye diseases through the fundamental inactivation of *VEGF-A* or by lowering the level of *VEGF-A* to less than a pathologic threshold.

An aspect provides a pharmaceutical composition for prevention and/or treatment of an eye disease, including a *VEGF-A* gene-inactivating agent.

The *VEGF-A* gene-inactivating agent may be at least one selected from the group consisting of proteins, nucleic acid molecules (DNA and/or RNA), and chemical drugs, all capable of inactivating a *VEGF-A* gene. In one embodiment, the *VEGF-A* gene-inactivating agent may comprise a Cas9 protein and a guide RNA targeting a *VEGF-A* gene.

Another aspect provides a method of preventing and/or treating an eye disease, comprising a step of inactivating a *VEGF-A* gene. The step of inactivating a *VEGF-A* gene may be carried out by a step of administering a *VEGF-A* gene-inactivating agent to a subject in need of prevention and/or treatment of an eye disease. The *VEGF-A* gene inactivation may be performed by RNA-guided genome surgery or RNA-guided genome editing. In this regard, the step of inactivating a *VEGF-A* gene is carried out by a step of administering a Cas9 protein and a guide RNA targeting a *VEGF-A* gene to a subject in need of prevention and/or treatment of an eye disease.

Another aspect provides the use of a *VEGF-A* gene-inactivating agent in prevention and/or treatment of an eye disease or in preparation of a therapeutic agent for an eye disease.

Another aspect provides a guide DNA for targeting a specific target site or target region of a *VEGFA* gene.

Another aspect provides a *VEGFA* gene-specific ribonucleoprotein (RNP) including a Cas9 protein and a guide RNA having a *VEGFA* gene-specific targeting sequence.

Another aspect provides a pharmaceutical composition including the guide RNA or the *VEGFA* gene-specific RNP.

Another aspect provides a method for treatment or prevention of an eye disease, including a step of administering the *VEGFA* gene-specific RNP to a subject in need of treatment and/or prevention of the eye disease.

### DETAILED DESCRIPTION

The present invention provides a technique for treating an eye disease, for example, an eye disease associated with the overexpression of VEGF-A, using a gene editing method.

The invention is set out in the appended set of claims. The references to the methods of treatment by therapy or surgery in examples 1 to 5 of this description are to be interpreted as references to compounds, pharmaceutical compositions and medicaments of the present invention for use in those methods.

An aspect provides a composition for the prevention and/or treatment of an eye disease, including a *VEGF-A* gene-inactivating agent. The *VEGF-A* gene-inactivating agent may be at least one selected from the group consisting of proteins, nucleic acid molecules (DNA and/or RNA), and chemical drugs all of which can inactivate a *VEGF-*A gene. In one embodiment, the *VEGF-A* gene-inactivating agent may include a Cas9 protein and a guide RNA targeting a *VEGF-A* gene.

Another aspect provides a method of preventing and/or treating an eye disease, comprising a step of in activating a *VEGF-A* gene. The step of inactivating a *VEGF-A* gene may be carried out by a step of administering a *VEGF-A* gene-inactivating agent to a subject in need of prevention and/or treatment of an eye disease. The *VEGF-A* gene inactivation may be performed by RNA-guided genome surgery or RNA-guided genome editing. In this regard, the step of inactivating a *VEGF-A* gene may be carried out by administering a Cas9 protein and a guide RNA targeting a *VEGF-A* gene to a subject in need of prevention and/or treatment of an eye disease. The method may further include a step of identifying the subject in need of prevention and/or treatment of an eye disease, prior to the administering step. The *VEGF-A* gene-inactivating agent may be administered in a pharmaceutically effective amount. The *VEGF-A* gene-inactivating agent may be administered via various routes, for example, by local administration to ocular lesions or by subretinal injection.

Another aspect provides the use of a *VEGF-A* gene-inactivating agent in prevention and/or treatment of an eye disease or in preparation of a therapeutic agent for an eye disease.

The *VEGF-A* gene to be targeted for inactivation may be located in an eye, for example, an eye having a neovascular eye disease , particularly, in a lesion site of a neovascular eye disease.

The *VEGF-A* gene inactivation may be at least one selected from the group consisting of:
(1) deletion of an entire sequence or a 1-50 bp or 1-40 bp long consecutive or inconsecutive partial sequence of the *VEGF-A* gene;
(2) substitution of 1-20, 1-15, or 1-10 consecutive or inconsecutive nucleotides in the *VEGF-A* gene with nucleotides different from those in a wild-type *VEGF-A* gene;
(3) insertion (addition) of 1-20, 1-15, or 1-10 nucleotides into the *VEGF-A* gene, the nucleotides to be inserted each being independently selected from among A, T, C, and G; and
(4) a combination thereof.

The *VEGF-A* gene-inactivating agent may be at least one selected from the group consisting of proteins, nucleic acid molecules (DNA and/or RNA), and chemical drugs, all of which can inactivate a *VEGF-A* gene. In accordance with one embodiment, the *VEGF-A* gene-inactivating agent may include a Cas9 protein and a guide RNA targeting a *VEGF-A* gene. In this regard, the *VEGF-A* gene inactivation may be performed by RNA-guided genome surgery or RNA-guided genome editing.

When carried out using a Cas9 protein, the *VEGF-A* gene inactivation may be at least one selected from the group consisting of:
(1) deletion of at least one nucleotide positioned in a 1-50 bp- or 1-40 bp-long consecutive or inconsecutive region, adjacent to a proto-spacer-adjacent motif (PAM) sequence for a Cas9 protein, in the *VEGF-A* gene;
(2) substitution of 1-20, 1-15, or 1-10 consecutive or inconsecutive nucleotides positioned in 1-50 bp- or 1-40 bp-long consecutive or inconsecutive region, adjacent to a PAM sequence for a Cas9 protein, in the *VEGF-A* gene with nucleotides different from those in a wild-type *VEGF-A* gene;
(3) insertion (addition) of 1-20, 1-15, or 1-10 nucleotides into a 1-50 bp- or 1-40 bp-long consecutive or inconsecutive region, adjacent to a PAM sequence for a Cas9 protein, in the *VEGF-A* gene, the nucleotides to be inserted each being independently selected from among A, T, C, and G; and
(4) a combination thereof.

The *VEGF-A* gene-inactivating agent may include a Cas protein or a coding gene thereof (DNA or mRNA); and a *VEGF-A* gene-specific guide RNA comprising a targeting sequence that binds specifically to a target size of a *VEGF-A* gene, or a coding DNA thereof.

The Cas9 protein and the *VEGF-A* gene-specific guide RNA may be in the form of:
(a) a complex in which the Cas9 protein is associated with the *VEGF-A* gene-specific guide RNA prior to administration to a body (or lesion) or cells (i.e., already assembled before administration), that is, in the form of ribonucleoprotein (RNP) (in this regard, transported in the form of RNP across cell membranes into cells or a body);
(b) a complex in which the Cas9 protein is associated with the *VEGF-A* gene-specific guide RNA following administration (delivery) into cells or a body by means of respective vectors carrying DNAs which respectively encode the Cas9 protein and the *VEGF-A* gene-specific guide RNA or one vector carrying both of the DNAs;
(c) a RNA mixture including an RNA (mRNA) coding for the Cas9 protein, and the VEGF-A gene-specific guide RNA; or
(d) a mixture of a recombinant vector carrying a gene (DNA) coding for the Cas9 protein and the VEGF-A gene-specific guide RNA (e.g., obtained by in vitro transcription).

In one embodiment, the RNA mixture may be included in a typical RNA carrier for delivery into cells or a body.

Therefore, the *VEGF-A* gene-inactivating agent may include:
(a) a complex in which the Cas9 protein is associated with the *VEGF-A* gene-specific guide RNA prior to administration to a body (or lesion) or cells (i.e., already assembled before administration), that is, ribonucleoprotein (RNP) (in this regard, transported in the form of RNP across cell membranes into cells or a body);
(b) a recombinant vector carrying together genes (DNA) coding respectively for a Cas protein and a *VEGF-A* gene-specific guide RNA, or separate recombinant vectors respectively carrying the genes (that is, a recombinant vector carrying a gene coding for a Cas protein and a recombinant vector carrying a DNA coding for a *VEGF-*A gene-specific guide RNA), or a recombinant cell anchoring the recombinant(s) thereat;
(c) an RNA mixture including an RNA (mRNA) coding for a Cas9 protein and a *VEGF-A* gene-specific guide RNA;
(d) a mixture of a recombinant vector carrying a gene (DNA) coding for a Cas9 protein, and a *VEGF-A* gene-specific guide RNA (i.e., obtained by in vitro transcription); or
(e) a combination thereof.

The administration of the *VEGF-A* gene-inactivating agent may be implemented by administering a recombinant vector carrying a gene (DNA) coding for a Cas9 protein and a recombinant vector carrying a DNA coding for a *VEGF-A* gene-specific guide RNA; an RNA (mRNA) coding for Cas9 protein, and a *VEGF-A* gene-specific guide RNA; or a recombinant carrying a gene (DNA) coding for a Cas9 protein, and a *VEGF-A* gene-specific guide RNA, simultaneously or sequentially irrespective of order.

Another aspect provides a guide RNA for targeting a predetermined target site or region. In one embodiment, the guide RNA may include a targeting sequence hybridizable with (i.e., a target sequence having a nucleic acid sequence complementary to) a nucleic acid sequence on one strand (e.g., a strand complementary to a strand on which a PAM sequence) of a predetermined target region in a *VEGFA* gene.

Another aspect provides a *VEGFA* gene-specific ribonucleoprotein (RNP) including a Cas9 protein and a guide RNA having a *VEGFA* gene-specific targeting sequence.

Another aspect provides a pharmaceutical composition including the guide RNA or the *VEGFA* gene-specific ribonucleoprotein (RNP). The pharmaceutical composition may be used for treating and/or preventing an eye disease such as macular degeneration (e.g., age-related macular degeneration (AMD)), retinopathy (e.g., diabetic retinopathy), etc.

Another aspect provides a method of treating or preventing an eye disease, including a step of administering the *VEGFA* gene-specific ribonucleoprotein (RNP) to a subject in need of treatment and/or prevention of the eye disease. The *VEGFA* gene-specific ribonucleoprotein (RNP) may be administered in a pharmaceutically effective amount, for example, via a topical route to an ocular lesion or by subretinal injection.

The eye disease may be an eye disease associated with the overexpression of a vascular endothelial growth factor (VEGF), for example, VEGF-A, as exemplified by a neovascular eye disease). The neovascular eye disease may be any eye disease that is caused by ocular neovascularization, for example, choroidal neovascularization (CNV) and may be selected from the group consisting of macular degeneration (e.g., age-related macular degeneration (AMD), myopic choroidal neovascularization, retinopathy (e.g., diabetic retinopathy), ischemic retinopathy, branch retinal vein occlusion, central retinal vein occlusion, and retinopathy of prematurity.

VEGF-A (vascular endothelial growth factor A) may be derived from mammals including primates such as humans, apes, and the like, and rodents such as rats, mice, etc. For example, it may be human VEGF-A (e.g., NCBI Accession No. NP_001020537, NP_001020538, NP_001020539, NP_001020540, NP_001020541, NP_001028928, NP_001165093, NP_001165094, NP_001165095, NP_001165096, NP_001165097, NP_001165098, NP_001165099, NP_001165100, NP_001165101, NP_001191313, NP_001191314, NP_001273973, NP_001303939, NP_003367, etc.), Mouse VEGF-A (NCBI Accession No. NP_001020421, NP_001020428, NP_001103736, NP_001103737, NP_001103738, NP_001273985, NP_001273986, NP_001273987, NP_001303970, NP_033531, etc.) .

The Cas9 protein may be derived (isolated) from, for example, *Streptococcus pyogenes.*

As used herein,
the term "target gene" refers to a gene to be targeted for gene editing *(VEGF-A* gene);
the term "target site" or "target region" refers to a gene site in a target gene *(VEGF-A* gene) in which Cas9 performs gene editing (cleavage, and deletion, addition and/or substitution of nucleotides), specifying a gene site with a maximum length of about 50 bp or 40 bp, adjacent to the 5' and/or 3' terminus of a PAM sequence recognized by Cas9 protein within the target gene *(VEGF-A* gene);
the term "target sequence" refers to a gene site of the target gene (*VEGF-A* gene), which can hybridize with a guide RNA, specifying a 17-23 bp, for example 20 bp-long nucleic acid sequence adjacent to the 5' or 3' end of a PAM sequence recognizable by Cas9 protein in the target gene; and
the term "targeting sequence" is a guide RNA region hybridizable with the target sequence in the target gene and may be a guide RNA region including 17-23, for example, 20 nucleotides.

In the present specification, the target sequence is represented by a nucleic acid sequence on the PAM sequence-retaining strand of the two DNA strands in a relevant gene region of the target gene *(VEGF-A* gene). Indeed, because the DNA strand to which the guide RNA binds is a complementary strand to the strand on which the PAM sequence is positioned, the targeting sequence in the guide RNA has the same nucleic acid sequence as the target sequence in the target gene *(VEGF-A* gene) except that T is changed into U in consideration of the RNA characteristic. Therefore, herein, a sequencing sequence of the guide RNA and a target sequence of the target gene *(VEGF-A* gene) are represented by the same nucleic acid sequence with the exception that T and U are interchanged.

When the Cas9 protein is derived from *Streptococcus pyogenes,* the PAM sequence is 5'-NGG-3' (wherein N is A, T, G, or C), and the target region is a gene region adjacent to the 5' terminus and/or 3' terminus of the 5'-NGG-3' sequence in the target gene *(VEGF-A* gene), and may be, for example, a gene region about 50 bp or about 40 bp long.

In this regard, the *VEGF-A* gene inactivation may be induced in a *VEGF-A* gene by:
a) deletion of at least one nucleotide from a nucleic acid sequence (target region) that is up to 50 bp or up to 40 bp in length and which is adjacent to the 5' and/or 3' terminus of a 5'-NGG-3' sequence (wherein N is A, T, C, or G),
b) substitution of at least one nucleotide (e.g., 1-20, 1-5, or 1-10 nucleotides) in a nucleic acid sequence (target site) which is up to 50 bp or up to 40 bp long and adjacent to the 5' and/or 3' terminus of a 5'-NGG-3' sequence, with a nucleotide different from a corresponding one in a wild-type gene,
c) insertion of at least one nucleotide (for example, 1-20, 1-5, or 1-10 nucleotides) into a nucleic acid sequence (target site) which is up to 50 bp or up to 40 bp long and adjacent to the 5' and/or 3' terminus of a 5'-NGG-3' sequence (for this, nucleotides to be inserted are each independently selected from among A, T, C, and G), or
d) a combination of two or more of a) to c).

The guide RNA may be at least one selected from the group consisting of a CRISPR RNA (crRNA), a trans-activating crRNA (tracrRNA), and a single guide RNA (sgRNA). In detail, the guide DNA may be a dual crRNA:tracrRNA complex in which a crRNA and a tracrRNA are combined with each other, or a single guide RNA (sgRNA) in which a crRNA or a part thereof is connected to a tracrRNA or a part thereof via an oligonucleotide linker.

The concrete sequence of the guide RNA may be suitably selected depending on kinds of Cas9 protein (that is, microorganisms from which the guide RNA is derived), and is a matter that a person skilled in the art could easily establish.

When a *Streptococcus* pyogenes-derived Cas9 protein is used, the crRNA may be represented by the following general formula 1:

5'-(N_{cas9})ₗ-(GUUUUAGAGCUA)-(X_{cas9})ₘ-3' (General Formula 1)

wherein,
N_{cas9} is a targeting sequence which is determined depending on a target sequence in a target gene *(VEGF-A* gene), I represents a number of nucleotides contained in the targeting sequence and may be an integer of 17 to 23, or 18 to 22, for example, 20;
a region including the 12 consecutive nucleotides (GUUUUAGAGCUA) (SEQ ID NO: 359) adjacent to the 3' terminus of the target sequence is an indispensable part of the crRNA,

X_{cas9} is a region including m nucleotides positioned at the 3' terminus of the crRNA (that is, adjacent to the 3' terminal of the indispensable part of the crRNA), and m may be an integer of 8 to 12, for example 11, and the m nucleotides, which may be the same or different, may each be independently selected from the group consisting of A, U, C, and G.

In one embodiment, X_{cas9} may include, but is not limited to, UGCUGUUUUG (SEQ ID NO: 360).

Further, the tracrRNA may be represented by the following general formula 2:

5'-(Y_{cas9})ₚ-(UAGCAAGUUAAAAUAAGGCUAGUCCGUUAUCAACUUGAAAAAGUGGCACCG AGUCGGUGC)-3' (General Formula 2)

wherein,
a region consisting of 60 nucleotides (UAGCAAGUUAAAAUAAGGCUAGUCCGUUAUCAACUUGAAAAAGUGGCACCG AGUCGGUGC) (SEQ ID NO: 361) is an indispensable part of the tracrRNA,
Y_{cas9} is a region including p nucleotides adjacent to the 5' terminus of the indispensable part of the tracrRNA, p may be an integer of 6 to 20, for example, 8 to 19, and the p nucleotides, which may be the same or different, may each be independently selected from the group consisting of A, U, C, and G.

The sgRNA may be in a form of a hairpin (stem-loop structure) in which a crRNA moiety including the targeting sequence and the indispensable part of the crRNA and a tracrRNA moiety including the indispensable part (60 nucleotides) of the tracrRNA is connected via an oligonucleotide linker (in this regard, the oligonucleotide linker accounts for the loop structure). In greater detail, the sgRNA has a hairpin structure in which a crRNA moiety including the targeting sequence and indispensable part of the crRNA and a tracrRNA moiety including the indispensable part of the tracrRNA are combined each other, with connection between the 3' terminus of the crRNA moiety and the 5' terminus of the tracrRNA moiety via an oligonucleotide linker.

In one embodiment, the sgRNA may be represented by the following general formula 3:

5'-(N_{cas9})ₗ-(GUUUUAGAGCUA)-(oligonucleotide linker)-(UAGCAAGUUAAAAUAAGGCUAGUCCGUUAUCAACUUGAAAAAGUGGCACCG AGUCGGUGC)-3' (General Formula 3)

wherein (N_{cas9})ₗ is a target sequence as defined above in General Formula 1.

The oligonucleotide linker included in the sgRNA may be a sequence consisting of three to five, for example, four nucleotides which may be the same or different and each be independently selected from the group consisting of A, U, C, and G.

The crRNA or the sgRNA may further include one to three guanines (G) at the 5' terminus (that is, the 5' terminus of the targeting sequence in the crRNA).

The tracrRNA or the sgRNA may further include a terminal region including five to seven uracil residues at the 3' terminus of the indispensable part (60 nt) of the tracrRNA.

In one embodiment, the target sequence in the target gene *(VEGF-A* gene) may be selected from the group consisting of:
Vegfa-1: 5'-CTCCTGGAAGATGTCCACCA-3' (SEQ ID NO: 1) (PAM sequence: GGG);
Vegfa-2: 5'-AGCTCATCTCTCCTATGTGC-3' (SEQ ID NO: 2) (PAM sequence: TGG);
Vegfa-3: 5'-GACCCTGGTGGACATCTTCC-3' (SEQ ID NO: 3) (PAM sequence: AGG);
Vegfa-4: 5'-ACTCCTGGAAGATGTCCACC-3' (SEQ ID NO: 4) (PAM sequence: AGG);
Vegfa-5: 5'- CGCTTACCTTGGCATGGTGG-3' (SEQ ID NO: 5) (PAM sequence: AGG);
Vegfa-6: 5'- GACCGCTTACCTTGGCATGG-3' (SEQ ID NO: 6) (PAM sequence: TGG);
Vegfa-7: 5'- CACGACCGCTTACCTTGGCA-3' (SEQ ID NO: 7) (PAM sequence: TGG); and
Vegfa-8: 5'- GGTGCAGCCTGGGACCACTG-3' (SEQ ID NO: 8) (PAM sequence: AGG).

These target sequences are well conserved among species and exist in, for example, both humans and rodents (e.g., mice). By way of example, the target sequence may include the nucleotide sequence of SEQ ID NO: 1 or SEQ ID NO: 2.

The target sequences are well conserved among mammals, for example, between human *VEGF-A* genes and mouse *VEGF-A* genes, and exhibit highly outstanding gene editing efficiency (e.g., indel frequency (%)) for on-target sites, and have 3 or less, 2 or less, 1, or no mismatching nucleotides in sites (off-target sites) other than the on-target sites. Thus, there is little or no probability of gene editing in sites other than the on-target sites, and the target sequences are of excellent safety (very low or almost no off-target effects)

On the basis of the outstanding editing efficiency and low off-target effect, an aspect of the present invention provides a composition for editing a *VEGF-A* gene, including the guide RNA or a DNA coding for the guide RNA, and a Cas9 protein or a gene (DNA or mRNA) coding for the Cas9 protein. The composition for editing a *VEGF-A* gene may include a ribonucleoprotein which contains the guide RNA and the Cas9 protein. In this regard, the ribonucleoprotein may be assembled prior to administration to a body or cells.

Herein, the targeting sequence of guide RNA hybridizable with the target region of the target gene means a nucleotide sequence having a sequence complementarity of 50 % or higher, 60 % or higher, 70 % or higher, 80 % or higher, 90 % or higher, 95 % or higher, 99 % or higher, or 100 % to a nucleotide sequence on a strand complementary to a DNA strand on which a target sequence is located (that is, a DNA strand on which a PAM sequence exists), and thus can complementarily bind to the nucleotide sequence on the complementary strand.

For example, the targeting sequence (N_{cas9})ₗ of crRNA or sgRNA may have the same sequence as one of the target sequences of SEQ ID NOS: 1 to 4 (provided that T is changed with U). This is, (N_{cas9})ₗ in crRNA or sgRNA may include a targeting sequence selected from among the sequences of SEQ ID NOS: 9 to 16:
*Vegfa-1:* 5'-CUCCUGGAAGAUGUCCACCA-3' (SEQ ID NO: 9);
Vegfa-2: 5'-AGCUCAUCUCUCCUAUGUGC-3' (SEQ ID NO: 10);
Vegfa-3: 5'-GACCCUGGUGGACAUCUUCC-3' (SEQ ID NO: 11);
Vegfa-4: 5'-ACUCCUGGAAGAUGUCCACC-3' (SEQ ID NO: 12);
*Vegfa-5:* 5'- CGCUUACCUUGGCAUGGUGG-3' (SEQ ID NO: 13);
*Vegfa-6:* 5'- GACCGCUUACCUUGGCAUGG-3' (SEQ ID NO: 14);
*Vegfa-7:* 5'- CACGACCGCUUACCUUGGCA-3' (SEQ ID NO: 15); and
*Vegfa-8:* 5'- GGUGCAGCCUGGGACCACUG-3' (SEQ ID NO: 16).

For example, the crRNA or the sgRNA may include SEQ ID NO: 9 or 10 as a targeting sequence.

In one embodiment, a modified RNA may be employed in order to solve the problem of lowering cell viability upon RNA delivery to bodies or cells. By way of example, an RNA which is modified so as to retain no phosphate-phosphate bonds at the 5 terminus thereof (e.g., no 5'-terminal triphosphate or diphosphate) may be used as a guide RNA. Another example is an sgRNA (e.g., chemically synthesized sgRNA) which contains one or more (e.g., one to five, or two to four) modified ribonucleic acids at the 5' and/or 3' terminus thereof. In this regard, the modification may be expressed as phosphorothioate or may include a modification at the 2' position of the ribose moiety (e.g., 2'-acetylation, 2'-methylation, etc.). In one embodiment, the modified sgRNA may include methylation (methyl group addition) at the 2'-O position of the ribose moiety on three nucleotides at each of the 5'-terminus and 3'-terminus and/or a phosphorothioate backbone modification.

Another aspect provides a guide RNA including a target sequence selected from among the sequences of SEQ ID NOS: 1 to 4.

In the method, the transduction of the guide RNA and the Cas9 protein into cells may be performed by directly introducing a pre-assembled complex (ribonucleoprotein) of the guide RNA and the Cas9 protein into cells with the aid of a conventional technique (e.g., electroporation, lipofection, etc.) into immune cells or one vector (e.g., plasmid, viral vector, etc.) carrying both a guide RNA-encoding DNA molecule and a Cas9 protein-encoding gene (DNA or mRNA) (or a gene having a sequence homology of 80 % or greater, 85 % or greater, 90 % or greater, 96 % or greater, 97 % or greater, 98 % or greater, or 99 % or greater thereto) or respective vectors carrying the DNA molecule or the gene into cells or through mRNA delivery.

In one embodiment, the vector may be a viral vector. The viral vector may be selected from the group consisting of negative-sense single-stranded viruses (e.g., influenza virus) such as retrovirus, adenovirus, parvovirus (e.g., adeno-associated virus (AAV)), corona virus, and orthomyxovirus; positive-sense single-stranded RNA viruses such as rhabdovirus (e.g., rabies virus and vesicular stomatitis virus), paramyxovirus (e.g., measles virus and sendai virus), alphavirus, and picornavirus; and double-stranded DNA viruses such as herpes virus (e.g., herpes simplex virus type 1 and 2, Epstein-Barr virus, cytomegalovirus), and adenovirus; poxvirus (e.g., vaccinia); fowlpox; and canarypox.

A vector carrying the Cas9 protein, the guide RNA, a ribonucleoprotein containing both of them, or at least one thereof may be delivered into a body or cells, using a suitable one of well-known techniques such as electroporation, lipofection, viral vector, nanoparticles, and PTD (protein translocation domain) fusion protein.

The Cas9 protein and/or guide RNA may further include a typically useful nuclear localization signal (NLS) for the intranuclear translocation of the Cas9 protein, the guide RNA, or the ribonucleoprotein containing both of them.

In the *VEGF-A* gene-specific ribonucleoprotein, the Cas9 protein may be isolated from microorganisms, or non-naturally occurring in a recombinant or chemically synthetic manner while the guide RNA may be produced in a recombinant manner or chemically.

*A VEGF-A* gene-inactivating agent or *VEGF-A* gene-specific ribonucleoprotein including a Cas9 protein and a gene (DNA or mRNA) encoding the protein, and a gene-specific guide RNA containing a targeting sequence specifically binding to a target region of a *VEGF-A* gene or a DNA encoding the RNA may be administered into a body via various routes including, but not limited to, a topical route and a subretinal route to a lesion of an eye disease (associated with *VEGF-A* gene overexpression).

Examples of a subject to be administered with the *VEGF-A* gene-inactivating agent or the *VEGF-A* gene-specific ribonucleoprotein include all animals, selected from mammals, for example, primates such as humans, apes, etc., and rodents such as mice, rats, etc., which suffer from or are at the risk of a *VEGF-A* gene overexpression-associated eye disease, cells (e.g., retinal pigment epithelial cells (RPE), RPE/choroid/scleral complex, etc.,) and tissues (eye tissues) isolated from the animals, and a culture of the cells or tissues.

The *VEGF-A* gene-inactivating agent or the *VEGF-A* gene-specific ribonucleoprotein may be administered in a "pharmaceutically effective amount" or contained in a "pharmaceutically effective amount" in a pharmaceutical composition. As used herein, the term "pharmaceutically effective amount" refers to an amount that can elicit a desirable effect, that is, a *VEGF-A* gene editing effect in an application region, and may be determined depending on various factors including the age, body weight, sex, and health state of the patient, the time and route of administration, excretion rate, and sensitivity to a drug.

### Effects of the Invention

Ensuring not only high gene editing efficiency, but also very low off-target effects, the *VEGF-A* gene editing technology suggested herein can perform gene editing effectively and safely whereby a *VEGF-A* protein level can be reduced to less than a pathological threshold, with the consequent long or permanent therapy of a *VEGF-A* overexpression-associated eye disease.

### BRIEF DESCRIPTION OF THE DRAWINGS

The above and other aspects, features and advantages of the present invention will be more apparent from the following detailed description taken in conjunction with the accompanying drawings, in which:
FIG. 1a shows target sequences in the *Vegfa*/*VEGFA* locus of mouse NIH3T3 and human ARPE-19 cells wherein PAM and sgRNA target sequences are shown in red and blue, respectively;
FIG. 1b shows mutations induced by the delivery of either Vegfa-specific Cas9 RNP containing Vegfa-1 sgRNA or a plasmid carrying an encoding sequence thereof in NIH3T3, as detected by the T7 endonuclease I (T7E1) assay;
FIG. 1c shows graphs of indel frequencies induced by the delivery of either the Vegfa-specific Cas9 RNP containing the Vegfa-1 sgRNA or a plasmid carrying an encoding sequence thereof in NIH3T3 and ARPE-19 cells;
FIG. 1d shows mutant DNA sequences induced by the Vegfa-specific Cas9 RNP containing the Vegfa-1 sgRNA at the *Vegfa*/*VEGFA* locus in NIH3T3 and ARPE-19 cells;
FIG. 1e is a graph showing indel frequencies induced by the delivery of the Vegfa-specific Cas9 RNP containing the Vegfa-1 sgRNA in confluent ARPE-19 cells;
FIG. 1f is a graph showing VEGFA mRNA levels in confluent ARPE-19 cells transfected with the Vegfa-specific Cas9 RNP containing the Vegfa-1 sgRNA;
FIG. 1g is a graph showing VEGFA protein levels in confluent ARPE-19 cells transfected with the Vegfa-specific Cas9 RNP containing the Vegfa-1 sgRNA;
FIG. 1h is a graph showing indel frequencies induced by the delivery of either Vegfa-specific Cas9 RNPs containing four sgRNAs (Vegfa-1 sgRNA, Vegfa-2 sgRNA, Vegfa-3 sgRNA, and Vegfa-4 sgRNA) or plasmids carrying encoding sequences thereof;
FIG. 2a shows confocal microscophic images of NIH3T3 cells 24 hours after transinfection with Cy3-labeled Cas9 RNP (a complex of Cy3-labeled Cas9 and Vegfa-1 sgRNA) or Cy3-labeled Cas9 alone (as a control);
FIG. 2b is a graph showing the proportion of Cy3 positive nuclei in total DAPI positive nuclei (100*[number of Cy3-positive nuclei]/[total number of DAPI-positive nuclei]) at 24 hours after transfection with Cy3-labeled Cas9 or Cy3-labeled Cas9 alone;
FIG. 2c shows mutations induced in NIH3T3 cells 24 hours post-transfection, as detected by T7 endonuclease 1 (T7E1) assay;
FIG. 2d is a graph showing indel frequencies induced in NIH3T3 cells 24 hours post-transfection;
FIG. 2e is a fluorescent image of an RPE flat-mount at day 3 after the injection of the Cy3-labeled Cas9 RNP into mouse eye, as observed under a fluorescence microscope;
FIG. 2f is a graph showing *in vivo* indel frequencies induced in genomic DNAs DNA isolated from the retinal pigment epithelium (RPE)/choroid/scleral complexes at day 3 after injection of the Cy3-labeled Cas9 RNP;
FIG. 2g shows Cas9 protein levels in RPE/choroid/scleral complexes 24 and 72 hours post-injection, as measured by Western blot analysis;
FIG. 2h is a fluorescent image showing the distribution of retinal pigment epithelium (RPE) in the RPE/choroid/scleral complex, as observed under a fluorescence microscope;
FIG. 2i shows Cas9 protein levels in RPE/choroid/scleral complexes 24 and 72 hours post-injection, as measured by Western blot analysis;
FIG. 3a is a schematic diagram illustrating the experimental procedure of Example 3;
FIG. 3b shows images visualizing laser-induced CNV stained with isolectin B4 (IB4) in C57BL/6J mice injected with the Rosa26-specific Cas9 RNP (as a control) or the Vegfa-specific Cas9 RNP at day 7 post-injection;
FIG. 3c is a graph showing the comparison of CNV areas in C57BL/6J mice injected with the Vegfa-specific Cas9 RNP and a control injection with the Rosa26-specific Cas9 RNP;
FIG. 3d is a graph showing Vegfa protein levels in CNV areas;
FIG. 3e is a graph of indel frequencies (%) at Vegfa target sites in the RPE complexes;
FIG. 3f is a graph of indel frequencies (%) at Rosa26 target sites in the RPE complexes;
FIG. 3g is an image of a laser-induced CNV structure in a cross-section sample, as visualized by hematoxylin & eosin staining;
FIG. 3h shows a CNV sample for use in mutation analysis through targeted deep sequencing;
FIG. 3i shows images of laser-induced CNV at day 7 after laser treatment;
FIG. 4a is a genome-wide Circos plot showing in vitro cleavage sites;
FIG. 4b shows sequence logos obtained using 42 sequences including 41 Digenome-capture sites and one On-target sequence;
FIG. 4c shows off-target sites and indel frequencies validated in human ARPE-19 cells;
FIG. 5 shows mutant DNA sequences induced by Vegfa-specific Cas9 RNPs (containing Vegfa-1 sgRNA) in mouse RPE, including (a) representative mutant sequences induced by the Vegfa-specific Cas9 RNP in RPE at day 3 post-injection; and (b) mutant DNA sequences in RPE containing laser-induced choroidal neovascularization (CNV) at day 7 post-injection;
FIG. 6 shows indel frequencies (%) in 20 potential off-target sites of mouse RPE;
FIG. 7a shows fluorescent cross-sectional images of the respective retinas obtained 7 days after injection of the Vegfa-specific Cas9 RNP from a normal mouse injected with the *Vegfa-specific* Cas9 *RNP* and a normal control mouse without injection of the Vegfa-specific Cas9 RNP;
FIG. 7b is a graph showing opsin positive areas (%);
FIG. 8 is a cleavage map of a pET28-NLS-Cas9 vector; and
FIG. 9 is a cleavage map of a pRG2 vector.

### EXAMPLES

Hereafter, the present invention will be described in detail by examples.

The following examples are intended merely to illustrate the invention and are not construed to restrict the invention.

### [REFERENCE EXAMPLE]

### 1. Preparation of Cas9 RNP

A purified Cas9 protein was purchased from ToolGen Inc., South Korea. sgRNAs were produced by in vitro transcription using a T7 polymerase (New England Biolabs) according to the manufacturer's protocol. In brief, templates for the sgRNAs were prepared by annealing and extending sets of two complementary oligonucleotides (see Table 1).

**Table 1**

| *In vitro* transcription templates encoding sgRNAs | |
|---|---|
| **sgRN A name** | **RGEN Target (5' to 3')** |
| *Vegfa* -1 (Forw ard) | GAAATTAATACGACTCACTATAGCTCCTGGAAGATGTCCACCAGTTTTAG AGCTAGAAATAGCAAG (SEQ ID NO: 17) |
| *Vegfa* -2 (Forw ard) | GAAATTAATACGACTCACTATAGAGCTCATCTCTCCTATGTGCGTTTTAG AGCTAGAAATAGCAAG (SEQ ID NO: 18) |
| *Vegfa* -3 (Forward) | GAAATTAATACGACTCACTATAGGACCCTGGTGGACATCTTCCGTTTTAG AGCTAGAAATAGCAAG (SEQ ID NO: 19) |
| *Vegfa* -4 (Forward) | GAAATTAATACGACTCACTATAGACTCCTGGAAGATGTCCACCGTTTTAG AGCTAGAAATAGCAAG (SEQ ID NO: 20) |
| Rosa 26 (Forward) | GAAATTAATACGACTCACTATAGGGCGGTCCTCAGAAGCCAGGGTTTTA GAGCTAGAAATAGCAAG (SEQ ID NO: 21) |
| Universal (Reverse) | AAAAAAGCACCGACTCGGTGCCACTTTTTCAAGTTGATAACGGACTAGC CTTATTTTAACTTGCTATTTCTAGCTCTAAAAC (SEQ ID NO: 22) |

(target sequences: underlined; indispensable parts of crRNA: bold; nucleotide linkers: italicized)

Each of the prepared sgRNA templates was added, together with a T7 RNA polymerase, to a reaction buffer (40 mM Tris-HCI, 20 mM MgCl₂, 2 mM spermidine, 1 mM DTT, pH7.9) including NTPs (Jena bioscience) and an RNase inhibitor (New England Biolabs), and incubated at 37°C for 16 hours for transcription. The sgRNA transcripts were incubated at 37°C for 30 min with DNase I (New England Biolabs). The sgRNAs were purified using RNeasy MinElute Cleanup Kit (Qiagen) and quantified using Nano drop (Thermo Fisher Scientific). The purified sgRNAs (65 µg) were incubated, together with CIP (Calf intestinal; 1000 units; Alkaline Phosphatase, New England Biolabs), at 37°C for 1 hour to remove the 3-phosphoric acid group. The resulting sgRNAs were again purified using RNeasy MinElute Cleanup Kit (Qiagen) and quantified using Nano drop (Thermo Fisher Scientific).

All the Cas9 proteins and the sgRNA stocks were assayed for cell viability and gene editing (indel) efficiency. Based on the assay, selection was made of Cas9 proteins and sgRNA stocks that exhibited high efficiency for use in *in vivo* eye injection.

### 2. Cas9 Protein Purification

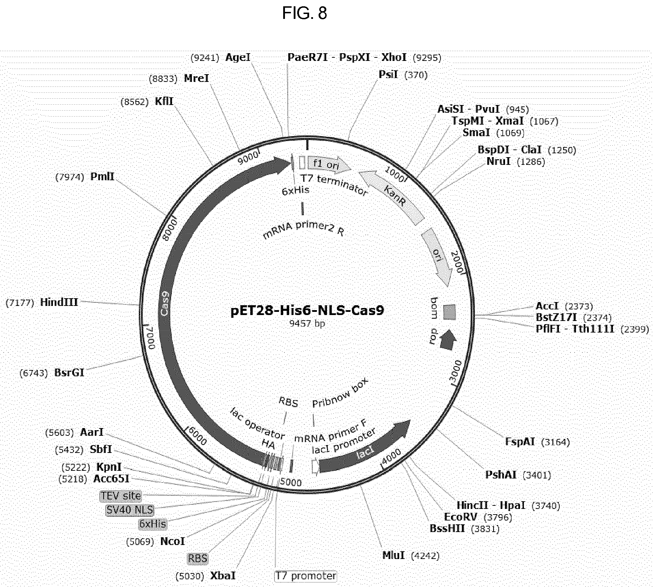

A pET28-NLS-Cas9 vector (FIG. 8; Cas9: *Streptococcus pyogenes* derived (SEQ ID NO: 358)) was transformed into the *E*. *coli* strain BL21 (DE3) which was then treated at 18°C for 12 hours with 0.5 mM isopropyl β-D-1-thiogalactopyranoside (IPTG) to induce the expression of the Cas9 protein. The E. coli cells were lysed by ultrasonication and centrifuged at 20,000g for 30 min. The soluble lysate thus obtained was mixed with Ni-NTA beads (Qiagen) and added with Cy3 dye (GE Healthcare) at a ratio of 1:10 (Cas9 protein: Cy3 dye molecule). The mixture was incubated overnight (12 hours or longer) at 4°C in a dark condition. Cy3-labeled Cas9 was eluted with elution buffer (50 mM Tris-HCI [pH 7.6], 150-500 mM NaCl, 10-25%(w/v) glycerol, 0.2 M imidazole), and dialyzed against dialyzing buffer (20 mM HEPES pH 7.5, 150 mM KCl, 1 mM DTT, 10%(w/v) glycerol). The purified Cy3-labeled Cas9 protein was concentrated using an Ultracel 100K cellulose column (Millipore). The Cy3-labeled Cas9 protein was measured for purity by SDS-PAGE. Cy3 labeling efficiency was determined by comparing absorption spectra of the Cas9 protein (280 nm) and the conjugated Cy3 dye molecule.

### 3. Cell culture and Transfection

Mouse NIH3T3 (ATCC^{®} CRL-1658^{™}) and ARPE-19 (human retinal pigment endothelial cell; ATCC^{®} CRL-2302^{™}) cell lines were cultured in Dulbecco's Modified Eagle Medium (DMEM) supplemented with 10%(v/v) BCS or FBS at 37°C under a humidified 5% CO₂ atmosphere (NIH3T3 and ARPE-19 cells had not been authenticated or tested for mycoplasma contamination). One day before transfection, NIH3T3 and ARPE-19 cells were seeded into 24-well plates at a cell density of 2x10⁴ cells/well, with each well containing 250 µl of an antibiotic-free growth medium.

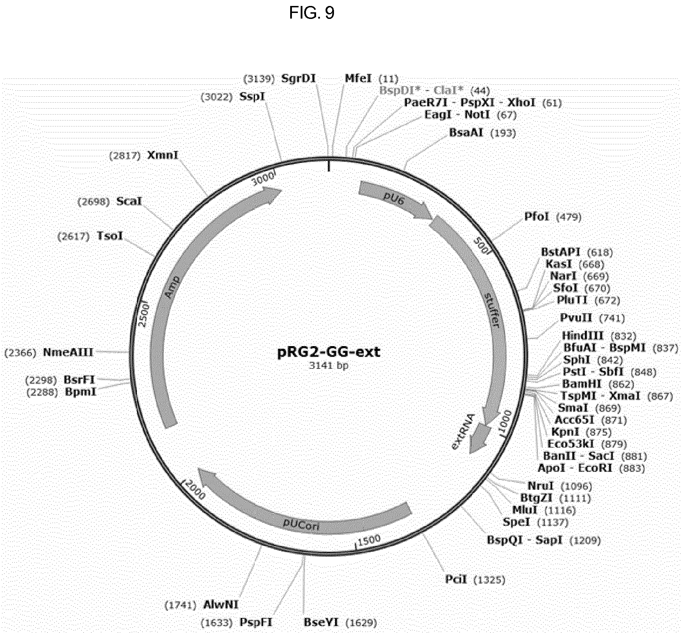

For plasmid delivery, the cells in the 24-well plates were transfected with a Cas9 (1 µg; *Streptococcus pyogenes* derived; coding sequence (4107bp): SEQ ID NO: 358) expression plasmid (pET vector (Addgene) used) and an sgRNA (1 µg; Example 1) expression plasmid (pRG2 vector (see FIG. 9) used), using Lipofectamine 2000 (Thermo Fisher Scientific) according to the manufacturer's protocol.

For RNP delivery, Cas9 protein (4 µg; Example 2) was incubated with sgRNA (2.25 µg; Example 1) at room temperature for 5 min, followed by adding 50 µl of Opti-MEM (Thermo Fisher Scientific) and 1 µl of Lipofectamine 2000 (Thermo Fisher Scientific). After 10 minutes, the RNP mixture was added to the 24-well plates and transfected into the cells. The cells were harvested 48 hours after transfection and subjected to T7E1 assay, targeted deep sequencing, and qPCR.

For VEGF-A expression in confluent RPE (human retinal pigment epithelial) cells, the prepared ARPE-19 cells were grown to confluency and then maintained in a 1% (v/v) FBS-supplemented DMEM/F12 to allow the formation of a polarized epithelial layer for experiments. ARPE-19 cells were added to 12-well plates and transfected with 8 µg of Cas9 protein, 4.5 µg of sgRNA, and 3 µl of Lipofectamine 2000. Two days after transfection, the transfection growth medium (DMEM + 1% (v/v) FBS) was replaced with 0. 5 ml of fresh serum-free medium. After 16 hours, cells and media were harvested and analyzed using targeted deep sequencing, qPCR, and ELISA.

### 4. Cy3-labeled Cas9 RNP imaging and counting

One day after transfection, cells were fixed in 4 % (w/v) PFA (paraformaldehyde) for 10 min at room temperature and then stained with 4', 6-diamidino-2-phenylindole (DAPI, 1 µg/ml, Sigma Aldrich) for 15 min at room temperature. The cells were visualized with a confocal microscope (LSM510, Carl Zeiss) at a magnification of x630. The scanning parameters were as follows: scaling (x = 0.14 µm/pixel, y = 0.14 µm/pixel, z = 1 µm/pixel), dimensions (x=1024, y=1024, z=6, channels: 3, 12-bit) (with objective C-Apochromat 63x/1.20W Korr UV-VIS-IR). Cy3 positive nuclei were counted using ZEN 2 software (black edition, Ver 10.0, Carl Zeiss). In order to quantify the frequency of Cy3 positive nuclei, a total number of cells and a number of cells with Cy staining in the nucleus were counted in a field of view at a magnification of x630 and an average percentage of Cy3 positive nuclei over four fields of view were calculated (n = 3).

### 5. T7E1 Assay

Genomic DNA was isolated from cells and tissues using a DNeasy Tissue Kit (Qiagen) according to the manufacturer's protocol. After target sites were amplified using PCR, the products were denatured and annealed using a thermal cycler. The primers used are summarized in Table 2, below.

**Table 2**

| | List of primers used for the T7E1 assay. | | | |
|---|---|---|---|---|
| | **1**^{st} **PCR** | | **2**^{nd} **PCR** | |
| **Target** | **Forward (5' to 3')** | **Reverse (5' to 3')** | **Forward (5' to 3')** | **Reverse (5' to 3')** |
| *Vegfa-1* (mouse) | CAAATCT GGGTGGC GATAGA (SEQ ID NO: 23) | AGATGGTCA AATCGTGGA GAG (SEQ ID NO: 24) | ACACTCTTT CCCTACACG ACGCTCTTC CGATCTCAA ATCTGGGTG GCGATAGA | GTGACTGG AGTTCAGA CGTGTGCT CTTCCGAT CTCCAGGG CTTCATCG |
| *Vegfa*-1 (human) | CATCGTG TGATCTCT GGAATGA A (SEQ ID NO: 27) | CCACCTGTT CCCAAAGTG TTA (SEQ ID NO: 28) | (SEQ ID NO: 25) ACACTCTTT CCCTACACG ACGCTCTTC CGATCTGTG GTGAAGTTC ATGGATGTC TA (SEQ ID NO: 29) | TTACA (SEQ ID NO: 26) GTGACTGG AGTTCAGA CGTGTGCT CTTCCGAT CTAAAGAT GCCCACCT GCAT (SEQ ID NO: 30) |

Annealed PCR products were incubated with T7 endonuclease I (ToolGen, Inc.) at 37°C for 25 min and analyzed by agarose gel electrophoresis.

### 6. Targeted deep sequencing

Using Phusion polymerase (Thermo Fisher Scientific), on-target and potential off-target regions were amplified from genomic DNA. The PCR amplicons were subjected to paired-end sequencing using Illumina MiSeq (LAS Inc. Korea). The primers used are listed in Tables 3 to 5:

**Table 3**

| List of primers used for targeted deep sequencing | | | | |
|---|---|---|---|---|
| | **1^{st} PCR** | | **2^{nd} PCR** | |
| **Target** | **Forward (5' to 3')** | **Reverse (5' to 3')** | **Forward (5' to 3')** | **Reverse (5' to 3')** |
| *Vegfa*-1 (mouse) | CAAATCTGG GTGGCGATA GA (SEQ ID NO: 23) | AGATGGTCA AATCGTGGA GAG (SEQ ID NO: 24) | ACACTCTTTCCC TACACGACGCT CTTCCGATCTCA AATCTGGGTGG CGATAGA (SEQ | GTGACTGGAG TTCAGACGTGT GCTCTTCCGAT CTCCAGGGCTT CATCGTTACA |
| | | | ID NO: 25) | (SEQ ID NO: 26) |
| *Vegfa*-2 (mouse) | ACCTATCCC TGCTCAGTA GAA (SEQ ID NO: 31) | CCCAAGAGA GGAAGCAAG AA (SEQ ID NO: 32) | ACACTCTTTCCC TACACGACGCT CTTCCGATCTAT CTGCTCCCTCC CTCTAC (SEQ ID NO: 33) | GTGACTGGAG TTCAGACGTGT GCTCTTCCGAT CTGTCCATCAC CATCACCACCA CCAC (SEQ ID NO: 34) |
| *Vegfa*-3 (mouse) | CAAATCTGG GTGGCGATA GA (SEQ ID NO: 23) | AGATGGTCA AATCGTGGA GAG (SEQ ID NO: 24) | ACACTCTTTCCC TACACGACGCT CTTCCGATCTCA AATCTGGGTGG CGATAGA (SEQ ID NO: 25) | GTGACTGGAG TTCAGACGTGT GCTCTTCCGAT CTCCAGGGCTT CATCGTTACA (SEQ ID NO: 26) |
| *Vegfa*-4 (mouse) | CAAATCTGG GTGGCGATA GA (SEQ ID NO: 23) | AGATGGTCA AATCGTGGA GAG (SEQ ID NO: 24) | ACACTCTTTCCC TACACGACGCT CTTCCGATCTCA AATCTGGGTGG CGATAGA (SEQ ID NO: 25) | GTGACTGGAG TTCAGACGTGT GCTCTTCCGAT CTCCAGGGCTT CATCGTTACA (SEQ ID NO: 26) |
| *Vegfa*-1 (human) | CATCGTGTG ATCTCTGGA ATGAA (SEQ ID NO: 27) | CCACCTGTT CCCAAAGTG TTA (SEQ ID NO: 28) | ACACTCTTTCCC TACACGACGCT CTTCCGATCTGT GGTGAAGTTCAT GGATGTCTA (SEQ ID NO: 29) | GTGACTGGAG TTCAGACGTGT GCTCTTCCGAT CTAAAGATGCC CACCTGCAT (SEQ ID NO: 30) |
| *Rosa26* (mouse) | CCAAAGTCG CTCTGAGTT GT (SEQ ID NO: 35) | TCGGGTGAG CATGTCTTTA ATC (SEQ ID NO: 36) | ACACTCTTTCCC TACACGACGCT CTTCCGATCTCC AAAGTCGCTCT GAGTTGT (SEQ ID NO: 37) | GTGACTGGAG TTCAGACGTGT GCTCTTCCGAT CTCTTTAAGCC TGCCCAGAAG A (SEQ ID NO: 38) |

**Table 4**

| List of primers used for targeted deep sequencing at potential off-target sites | | | | |
|---|---|---|---|---|
| | **1^{st} PCR** | | **2^{nd} PCR** | |
| **No.** | **Forward (5' to 3')** | **Reverse (5' to 3')** | **Forward (5' to 3')** | **Reverse (5' to 3')** |
| OT1 | TCTGTCTGTC TCCAGACATT TG (SEQ ID NO: 39) | GTCCTGCTTCT ATCCTGCTTTA (SEQ ID NO: 40) | ACACTCTTTCC CTACACGACG CTCTTCCGATC TGTGATCAGCT GACTTCCAGTT C (SEQ ID NO: 41) | GTGACTGGAG TTCAGACGTGT GCTCTTCCGAT CTCTCCACAAC TCAAGTCCCAT TAC (SEQ ID NO: 42) |
| OT2 | GACGTGAGA GTGAGCAGTT TAT (SEQ ID NO: 43) | ACAGCACCCA GATTGTCTTC (SEQ ID NO: 44) | ACACTCTTTCC CTACACGACG CTCTTCCGATC TCCTTGTGTCC TTTGATGCTCT (SEQ ID NO: 45) | GTGACTGGAG TTCAGACGTGT GCTCTTCCGAT CTAAGGTCTGC ACCATGAATCC (SEQ ID NO: 46) |
| OT3 | GCTGTGCTCA AGACCAACAA (SEQ ID NO: 47) | CCGGTTCTGTA CTGGTGTCT (SEQ ID NO: 48) | ACACTCTTTCC CTACACGACG CTCTTCCGATC TTTGCCTACCT CCACCTTCT (SEQ ID NO: 49) | GTGACTGGAG TTCAGACGTGT GCTCTTCCGAT CTAGATGCTGC CCTACATGAAC (SEQ ID NO: 50) |
| OT4 | GTAGGCTCAA CAGCTCTTTC T (SEQ ID NO: 51) | CATAGTGTGAG TGGTACTGGTG (SEQ ID NO: 52) | ACACTCTTTCC CTACACGACG CTCTTCCGATC TCCTGAGCTTC CTCTGTCCTAA T (SEQ ID NO: 53) | GTGACTGGAG TTCAGACGTGT GCTCTTCCGAT CTGCCACTGCT TCTCCTCTCTA T (SEQ ID NO: 54) |
| OT5 | GCCCAAAGTA GCAGGTGATT A (SEQ ID NO: 55) | CTCAGGCTGTA ACTGACGATAT G (SEQ ID NO: 56) | ACACTCTTTCC CTACACGACG CTCTTCCGATC TACAGGATGCA AGTCCACATC (SEQ ID NO: 57) | GTGACTGGAG TTCAGACGTGT GCTCTTCCGAT CTCATTCTTCA CAGGGCCATC A (SEQ ID NO: 58) |
| OT6 | AGAAGCTAAG GAGCCCAATT T (SEQ ID NO: 59) | TACTTTGCCAA GCCCATGT (SEQ ID NO: 60) | ACACTCTTTCC CTACACGACG CTCTTCCGATC TGCCTTCTCTC TTGGCTGTAA (SEQ ID NO: 61) | GTGACTGGAG TTCAGACGTGT GCTCTTCCGAT CTGAACCTACT CTCATCGTGCT AC (SEQ ID NO: 62) |
| OT7 | GAGGAGCCC AAGTATATCA CAG (SEQ ID NO: 63) | GGTCACCATAG CTACAAGAGAG (SEQ ID NO: 64) | ACACTCTTTCC CTACACGACG CTCTTCCGATC TAAGGCTCCAT TAGCCTCTTC (SEQ ID NO: 65) | GTGACTGGAG TTCAGACGTGT GCTCTTCCGAT CTCTGTCATGG TGCACATCATT C (SEQ ID NO: 66) |
| OT8 | CCCTGCAGCA TTCTCTGTAT (SEQ ID NO: 67) | GACCCAGTGTA TTGTGGGTAG (SEQ ID NO: 68) | ACACTCTTTCC CTACACGACG CTCTTCCGATC TTGACAAGCCT GACAGTTCATC (SEQ ID NO: 69) | GTGACTGGAG TTCAGACGTGT GCTCTTCCGAT CTGGCTGATG GTGAGCAGAA A (SEQ ID NO: 70) |
| OT9 | CTGGAACCAG AGTCATAGAT AGTTG (SEQ ID NO: 71) | TCTGAAGCACA CACCAGAAG (SEQ ID NO: 72) | ACACTCTTTCC CTACACGACG CTCTTCCGATC TCAAGATACCA AAGCAGGTGTT | GTGACTGGAG TTCAGACGTGT GCTCTTCCGAT CTGAAGCAGTT CAGAGGTCTAT |
| | | | C (SEQ ID NO: 73) | GT (SEQ ID NO: 74) |
| OT10 | CTAGAAGAAG GCAGAGGGA GTA (SEQ ID NO: 75) | AGGAGGGACA GACTGGTATAA A (SEQ ID NO: 76) | ACACTCTTTCC CTACACGACG CTCTTCCGATC TCACAGCGAG CCAGAATACA (SEQ ID NO: 77) | GTGACTGGAG TTCAGACGTGT GCTCTTCCGAT CTCTGTGCTAC CTGATCTACTC AAC (SEQ ID NO: 78) |
| OT11 | GTGTGAATGG AGGCGAAATT G (SEQ ID NO: 79) | GCAGCTGAGA AGCTAAGGAAT A (SEQ ID NO: 80) | ACACTCTTTCC CTACACGACG CTCTTCCGATC TTACATAAAGT CCCTGCAACCT G (SEQ ID NO: 81) | GTGACTGGAG TTCAGACGTGT GCTCTTCCGAT CTTTACCAGGA CTCTAGTGAGT GG (SEQ ID NO: 82) |
| OT12 | TAGTACCTGC CCACCAGATA G (SEQ ID NO: 83) | GGGCACTTCTT CAATGCTTTAC (SEQ ID NO: 84) | ACACTCTTTCC CTACACGACG CTCTTCCGATC TCTCCTGACCA GTGTTCTGTAA T (SEQ ID NO: 85) | GTGACTGGAG TTCAGACGTGT GCTCTTCCGAT CTAAACCTCGA GTAGGAAGGG A (SEQ ID NO: 86) |
| OT13 | CCCACTGAG GTTGTATCAG TTC (SEQ ID NO: 87) | GATCCAATGGC TTTGCACATAC (SEQ ID NO: 88) | ACACTCTTTCC CTACACGACG CTCTTCCGATC TAAAGAAGACC AGTGAAGGACT G (SEQ ID NO: 89) | GTGACTGGAG TTCAGACGTGT GCTCTTCCGAT CTAGTCTGATG ACCCGAGTTCT A (SEQ ID NO: 90) |
| OT14 | TCTATATAGG CAGGTTATGA AAGCA (SEQ | AACCAGGACAT ATGTGGTAGAA A (SEQ ID NO: | ACACTCTTTCC CTACACGACG CTCTTCCGATC | GTGACTGGAG TTCAGACGTGT GCTCTTCCGAT |
| | ID NO: 91) | 92) | TAATGGCCTTC TGGGAAAGT (SEQ ID NO: 93) | CTCTGAGTCTG AGAGCTTGTAG TG (SEQ ID NO: 94) |
| OT15 | CACAGACAGT CGCCTTCAAT (SEQ ID NO: 95) | TGGAAGCCTTA ACAGGTCAATA A (SEQ ID NO: 96) | ACACTCTTTCC CTACACGACG CTCTTCCGATC TGCCTTCAATG AATCTCCCTTT G (SEQ ID NO: 97) | GTGACTGGAG TTCAGACGTGT GCTCTTCCGAT CTGCTTCATTG GCAGCACTTAC (SEQ ID NO: 98) |
| OT16 | GGAAGATCAG CAGTCTCAAC TAA (SEQ ID NO: 99) | CACATTACCTC AAAGCTGTTTC TT (SEQ ID NO: 100) | ACACTCTTTCC CTACACGACG CTCTTCCGATC TCTCAGTGACA GAGACTCACCT A (SEQ ID NO: 101) | GTGACTGGAG TTCAGACGTGT GCTCTTCCGAT CTGTGGTGACA TGGCTGTATCT T (SEQ ID NO: 102) |
| OT17 | CTTCCACCGG GTATTTCCTA TC (SEQ ID NO: 103) | TCCCAGAGAG AGTTAGGTTAA GA (SEQ ID NO: 104) | ACACTCTTTCC CTACACGACG CTCTTCCGATC TAGATGAATGA GCACCAGAGA AA (SEQ ID NO: 105) | GTGACTGGAG TTCAGACGTGT GCTCTTCCGAT CTAGACAAGAA AGGGCAGTAA GAA (SEQ ID NO: 106) |
| OT18 | CCTGGGAACA ACAGCCATAA (SEQ ID NO: 107) | GAACATTGGGT AGGTGAGGAA G (SEQ ID NO: 108) | ACACTCTTTCC CTACACGACG CTCTTCCGATC TTCTCTGTTGA GGTGGGATTT G (SEQ ID NO: 109) | GTGACTGGAG TTCAGACGTGT GCTCTTCCGAT CTGTACTGCTT GAGGAGCTTG T (SEQ ID NO: 110) |
| OT19 | TGAGCCAGTC | TCCCTCCTGTT | ACACTCTTTCC | GTGACTGGAG |
| | CATTCATTCC (SEQ ID NO: 111) | CTTCTCTTCT (SEQ ID NO: 112) | CTACACGACG CTCTTCCGATC TTTGGGACAAG TGTACAGAGAA C (SEQ ID NO: 113) | TTCAGACGTGT GCTCTTCCGAT CTACCTTCACC TACAGAGAAGA GA (SEQ ID NO: 114) |
| OT20 | CCCACAAACC AAGAACAACA A (SEQ ID NO: 115) | CAGTGTTAAGT GCCTCTGTAGA T (SEQ ID NO: 116) | ACACTCTTTCC CTACACGACG CTCTTCCGATC TCAGAAGGGC GGCATCAG (SEQ ID NO: 117) | GTGACTGGAG TTCAGACGTGT GCTCTTCCGAT CTTTTAGTCTC TGGTTTCCACC T (SEQ ID NO: 118) |

**Table 5**

| List of primers used for targeted deep sequencing at potential off-target sites captured by Digenome-seq | | | | |
|---|---|---|---|---|
| | **1^{st} PCR** | | **2^{nd} PCR** | |
| **No.** | **Forward (5' to 3')** | **Reverse (5' to 3')** | **Forward (5' to 3')** | **Reverse (5' to 3')** |
| OT1 | ATGGAGCTTG CATTTTAACA (SEQ ID NO: 119) CAAACTGTCA GTGAGCCAAT AC (SEQ ID NO: 123) | CTTTTTCCCG TGATCCTCA (SEQ ID NO: 120) GAAGTGATCC TCCTCTCAATA CC (SEQ ID NO: 124) | ACACTCTTTCC CTACACGACG CTCTTCCGATC TATGGAGCTT GCATTTTAACA (SEQ ID NO: 121) ACACTCTTTCC CTACACGACG CTCTTCCGATC TCAGGAAGTC | GTGACTGGAG TTCAGACGTGT GCTCTTCCGAT CTGCTGGCTT ATTTCATCATT TAG (SEQ ID NO: 122) GTGACTGGAG TTCAGACGTGT GCTCTTCCGAT CTCATCCATCC |
| OT2 | CAAACTGTCA GTGAGCCAAT AC (SEQ ID NO: 123) | GAAGTGATCC TCCTCTCAATA CC (SEQ ID NO: 124) | ACACTCTTTCC CTACACGACG CTCTTCCGATC TCAGGAAGTC | GTGACTGGAG TTCAGACGTGT GCTCTTCCGAT CTCATCCATCC |
| | | | AAGCAGGAAG A (SEQ ID NO: 125) | ATTCATAACTT TGGA (SEQ ID NO: 126) |
| OT3 | GTCCAATACTC TAAGCCTCAGT T (SEQ ID NO: 127) | ACCAGCACCA CACTATCTATT T (SEQ ID NO: 128) | ACACTCTTTCC CTACACGACG CTCTTCCGATC TATACCTAGTT TGTAGGGTTG TT (SEQ ID NO: 129) | GTGACTGGAG TTCAGACGTGT GCTCTTCCGAT CTGCACCACA CTATCTATTTC TGTTAT (SEQ ID NO: 130) |
| OT4 | ACACTATGATC TTTCCCTGCAA (SEQ ID NO: 131) | CAGAAACCCT GAAGTCTTGAA TTG (SEQ ID NO: 132) | ACACTCTTTCC CTACACGACG CTCTTCCGATC TTCTTTCCCTG CAAAGAAGTAA GA (SEQ ID NO: 133) | GTGACTGGAG TTCAGACGTGT GCTCTTCCGAT CTGTCTCATTG TCCAGAACTGT GT (SEQ ID NO: 134) |
| OT5 | GGGCAGAAAG GACAGAAACT (SEQ ID NO: 135) | GGAGAAACTG AAACCAGGAG AA (SEQ ID NO: 136) | ACACTCTTTCC CTACACGACG CTCTTCCGATC TCGTAACAGC ACCTTGGTCAT (SEQ ID NO: 137) | GTGACTGGAG TTCAGACGTGT GCTCTTCCGAT CTCTGAAACCA GGAGAAGTGT AGTC (SEQ ID NO: 138) |
| OT6 | AGTAGGTGGG AGGGTTCTTAT (SEQ ID NO: 139) | CACCATCTCTG TGTCTCATCTG (SEQ ID NO: 140) | I ACACTCTTTCC CTACACGACG CTCTTCCGATC TAGAAACAGG CATCTGGAGA AC (SEQ ID NO: 141) | GTGACTGGAG TTCAGACGTGT GCTCTTCCGAT CTTTCAGCATA GTCTTGCTCGT C (SEQ ID NO: 142) |
| OT7 | TAAGCCTGGC CTGTCTCTT | AGAGCAGGAC GTGGTGAG | ACACTCTTTCC CTACACGACG | GTGACTGGAG TTCAGACGTGT |
| | (SEQ ID NO: 143) | (SEQ ID NO: 144) | CTCTTCCGATC TTCTCTTCCTG GGACCCT (SEQ ID NO: 145) | GCTCTTCCGAT CTATACCTAGG AATGCAGAAC AAG (SEQ ID NO: 146) |
| OT8 | GGGATTGCAC TTAGGTTCTTC T (SEQ ID NO: 147) | CTATGCGGTC TCTTGTGCTAA T (SEQ ID NO: 148) | ACACTCTTTCC CTACACGACG CTCTTCCGATC TGGTCAGGTG GGTAATGATTT CTG (SEQ ID NO: 149) | GTGACTGGAG TTCAGACGTGT GCTCTTCCGAT CTCTAATCTGC CTTATGTAATG GGTTCT (SEQ ID NO: 150) |
| OT9 | GACTCCTCTGT GGAAAGAGC (SEQ ID NO: 151) | AGGACTCCAG TGCTGAGCAC (SEQ ID NO: 152) | ACACTCTTTCC CTACACGACG CTCTTCCGATC TTCCTCTGTGG AAAGAGCCT (SEQ ID NO: 153) | GTGACTGGAG TTCAGACGTGT GCTCTTCCGAT CTACACCGTCT CTCCTTTGTGC (SEQ ID NO: 154) |
| OT10 | AGGGACCGTA TCAGATATTGT TAATC (SEQ ID NO: 155) | TCCAATGTATT GCAGCCATCT (SEQ ID NO: 156) | ACACTCTTTCC CTACACGACG CTCTTCCGATC TAATCAATCCT TGTGCAGCTTA ATG (SEQ ID NO: 157) | GTGACTGGAG TTCAGACGTGT GCTCTTCCGAT CTCAGCCATCT TGCCCTTTGA (SEQ ID NO: 158) |
| OT11 | CATTGAGGAA CCTCACCTTCT AT (SEQ ID NO: 159) | ATGAATGTCTT GGTACTGTCC TC (SEQ ID NO: 160) | ACACTCTTTCC CTACACGACG CTCTTCCGATC TGGAAGAGGT GTATTAGGCC ATT (SEQ ID NO: 161) | GTGACTGGAG TTCAGACGTGT GCTCTTCCGAT CTCCTCTTCTC TCTTGCTTCAT CTC (SEQ ID NO: 162) |
| OT12 | CAAAGCAGCT CCTCTTCCTC (SEQ ID NO: 163) | CAGTGCCTTTC AGTGAACCT (SEQ ID NO: 164) | ACACTCTTTCC CTACACGACG CTCTTCCGATC TTCTGGGTATA GAGACCATGA CA (SEQ ID NO: 165) | GTGACTGGAG TTCAGACGTGT GCTCTTCCGAT CTCACAGCCT GAGATAATGAT AGAGAG (SEQ ID NO: 166) |
| OT13 | GGAGTCGTAC CCTGGTTTATT T (SEQ ID NO: 167) | GAAGCATTGTT CCACCTTAACC (SEQ ID NO: 168) | ACACTCTTTCC CTACACGACG CTCTTCCGATC TGGGATAGAA GATTAGGCAG AGTATG (SEQ ID NO: 169) | GTGACTGGAG TTCAGACGTGT GCTCTTCCGAT CTTGCATGTTT GAAAGGATGA GC (SEQ ID NO: 170) |
| OT14 | CTCTCAGACC CTACTCACCTA T (SEQ ID NO: 171) | CACTGGAAGT ACCTGTGGAA G (SEQ ID NO: 172) | ACACTCTTTCC CTACACGACG CTCTTCCGATC TAGACCCTACT CACCTATATCC TTT (SEQ ID NO: 173) | GTGACTGGAG TTCAGACGTGT GCTCTTCCGAT CTTACCTGTG GAAGCAGGAG A (SEQ ID NO: 174) |
| OT15 | GGCCATCCTC AAAGACATGAA (SEQ ID NO: 175) | TCTCAAACTCC CGACCTCA (SEQ ID NO: 176) | ACACTCTTTCC CTACACGACG CTCTTCCGATC TGCATTTCTAT TTATTCATCTC CCACAG (SEQ ID NO: 177) | GTGACTGGAG TTCAGACGTGT GCTCTTCCGAT CTCTGGGATTA CAGGCGTGAG (SEQ ID NO: 178) |
| OT16 | AGAAGTTTCAG GATGACAGAT CC (SEQ ID NO: 179) | CAATCCACATC TGCGTGTTTC (SEQ ID NO: 180) | ACACTCTTTCC CTACACGACG CTCTTCCGATC TAGAAGTTTCA GGATGACAGA | GTGACTGGAG TTCAGACGTGT GCTCTTCCGAT CTCAATCCACA TCTGCGTGTTT |
| | | | TCC (SEQ ID NO: 181) | C (SEQ ID NO: 182) |
| OT17 | TGACTCATTGT GAATGCCTTTA TTC (SEQ ID NO: 183) | GAGTTGGGTT CTCTGCAACT (SEQ ID NO: 184) | ACACTCTTTCC CTACACGACG CTCTTCCGATC TTATAGAGTCT AGATTAGCAGT AGAGC (SEQ ID NO: 185) | GTGACTGGAG TTCAGACGTGT GCTCTTCCGAT CTAAGTCTTAT CTGATACATG GATACC (SEQ ID NO: 186) |
| OT18 | TGCAGCTCTG GACAGGAA (SEQ ID NO: 187) | GGTGGGTTTC ACCATCCTC (SEQ ID NO: 188) | ACACTCTTTCC CTACACGACG CTCTTCCGATC TGGGTGATTC CCTCTGTGG (SEQ ID NO: 189) | GTGACTGGAG TTCAGACGTGT GCTCTTCCGAT CTCCATCCTCC TGCCCTCT (SEQ ID NO: 190) |
| OT19 | GACAGCACTT AGGGATGATG AA (SEQ ID NO: 191) | GATGGAGCTG CCCAAGAAA (SEQ ID NO: 192) | ACACTCTTTCC CTACACGACG CTCTTCCGATC TGGGATGATG AATGGCTGGA T (SEQ ID NO: 193) | GTGACTGGAG TTCAGACGTGT GCTCTTCCGAT CTCTTCTCCAT GTAGGTGCCT T (SEQ ID NO: 194) |
| OT20 | CCTGAGAACA AGGAGTGTCA AG (SEQ ID NO: 195) | CCATGGAATG CCCAGATAGTT (SEQ ID NO: 196) | ACACTCTTTCC CTACACGACG CTCTTCCGATC TGTTGATATCC CAGCTTAAGC AATC (SEQ ID NO: 197) | GTGACTGGAG TTCAGACGTGT GCTCTTCCGAT CTTTAAACATC ATTTCTGGCAC GTC (SEQ ID NO: 198) |
| OT21 | AGCTATTGCTG TCAATCTCTTA CT (SEQ ID NO: | TACCCAGTCTC AGGTAGTTCTT (SEQ ID NO: | ACACTCTTTCC CTACACGACG CTCTTCCGATC | GTGACTGGAG TTCAGACGTGT GCTCTTCCGAT |
| | 199) | 200) | TTGCTGTCAAT CTCTTACTGTA ACTA (SEQ ID NO: 201) | CTTAGCAATGC GAGAACAGAC TAA (SEQ ID NO: 202) |
| OT22 | TGCCACACAT CCCATCATATC (SEQ ID NO: 203) | CAGCAGACAC AGACTCACAA (SEQ ID NO: 204) | ACACTCTTTCC CTACACGACG CTCTTCCGATC TCAACATGAAA TGCCAGAGTC AAA (SEQ ID NO: 205) | GTGACTGGAG TTCAGACGTGT GCTCTTCCGAT CTCCCATTCAA GTTGCAATCAC TATC (SEQ ID NO: 206) |
| OT23 | TCCTGAAAGAA GGGATAAGGT AAG (SEQ ID NO: 207) | TGAGGATGGG TTTCGGTAAAT (SEQ ID NO: 208) | ACACTCTTTCC CTACACGACG CTCTTCCGATC TATAAGGTAAG CTCAGCCTGT C (SEQ ID NO: 209) | GTGACTGGAG TTCAGACGTGT GCTCTTCCGAT CTGTTTCAACA TGAAGGCAAG GAG (SEQ ID NO: 210) |
| OT24 | CAAGAAGGGT GTTAGGTTATG AAAG (SEQ ID NO: 211) | ACAGTCAACC CTTAAGGAAG AG (SEQ ID NO: 212) | ACACTCTTTCC CTACACGACG CTCTTCCGATC TGGGTGTTAG GTTATGAAAGT TTAAGG (SEQ ID NO: 213) | GTGACTGGAG TTCAGACGTGT GCTCTTCCGAT CTAAGGAAGA GTTGTCTTCAC TCG (SEQ ID NO: 214) |
| OT25 | CTTTCACAGCC AGTCACAAATA AA (SEQ ID NO: 215) | CTCACACTCTA GGAAACAGAT GATAG (SEQ ID NO: 216) | ACACTCTTTCC CTACACGACG CTCTTCCGATC TCAATCCACTC AGACTACAGA GAAA (SEQ ID NO: 217) | GTGACTGGAG TTCAGACGTGT GCTCTTCCGAT CTAGACAGGA GTGTTCTCCAA ATC (SEQ ID NO: 218) |
| OT26 | GTGAGCCAAG | CTCTCAGCAA | ACACTCTTTCC | GTGACTGGAG |
| | ATCACACCAT (SEQ ID NO: 219) | GAAGGCAGAT T (SEQ ID NO: 220) | CTACACGACG CTCTTCCGATC TAGATCACACC ATTGCACTCC (SEQ ID NO: 221) | TTCAGACGTGT GCTCTTCCGAT CTGCCAGATC AGTGTCTGCTA AA (SEQ ID NO: 222) |
| OT27 | GGACACGCTG AGTCAAAGTT (SEQ ID NO: 223) | CCTTTCCTTCG TGCTGATTGA (SEQ ID NO: 224) | ACACTCTTTCC CTACACGACG CTCTTCCGATC TGCAACCACG TCGACAATACA (SEQ ID NO: 225) | GTGACTGGAG TTCAGACGTGT GCTCTTCCGAT CTGGTGGAAG TGACAAGCAA GTTA (SEQ ID NO: 226) |
| OT28 | CCCAACAATTC CTTCTTTGAGC (SEQ ID NO: 227) | TCTGCTATTAG AGGAGGCTAG AA (SEQ ID NO: 228) | ACACTCTTTCC CTACACGACG CTCTTCCGATC TCAATTCCTTC TTTGAGCTCAC TAT (SEQ ID NO: 229) | GTGACTGGAG TTCAGACGTGT GCTCTTCCGAT CTGAGGCTAG AACAACCTTG GA (SEQ ID NO: 230) |
| OT29 | GGGCAAATCC ATAACCCAGAA TA (SEQ ID NO: 231) | AGGCGATGCA TGAGCTTAAA (SEQ ID NO: 232) | ACACTCTTTCC CTACACGACG CTCTTCCGATC TGGGCAAATC CATAACCCAG A (SEQ ID NO: 233) | GTGACTGGAG TTCAGACGTGT GCTCTTCCGAT CTGTAGCTAAT CTGGCTACCA TCAC (SEQ ID NO: 234) |
| OT30 | ATTGGCTGGC ACACAGTAG (SEQ ID NO: 235) | CCCAGGATCT AGCAAACATTC A (SEQ ID NO: 236) | ACACTCTTTCC CTACACGACG CTCTTCCGATC TTGAATGAATG AAGGAAAGAA TGGG (SEQ ID | GTGACTGGAG TTCAGACGTGT GCTCTTCCGAT CTGCAAACATT CATCTTTCGAG CTA (SEQ ID |
| | | | NO: 237) | NO: 238) |
| OT31 | CACTTCTCGC CTTTGACCTT (SEQ ID NO: 239) | TGGCTGTGCT CACTTTACTG (SEQ ID NO: 240) | ACACTCTTTCC CTACACGACG CTCTTCCGATC TAGAGGAGGA AACTGGAGCT TA (SEQ ID NO: 241) | GTGACTGGAG TTCAGACGTGT GCTCTTCCGAT CTACTTTACTG CCACCAGTGC (SEQ ID NO: 242) |
| OT32 | ATCTTCCACAG GTGCAAATCT (SEQ ID NO: 243) | TTGCCTATGG CTGCCTTG (SEQ ID NO: 244) | ACACTCTTTCC CTACACGACG CTCTTCCGATC TCTGGTCATTC TCTTCCGTCAA A (SEQ ID NO: 245) | GTGACTGGAG TTCAGACGTGT GCTCTTCCGAT CTAACAGTATG GGCCTGAAAA G (SEQ ID NO: 246) |
| OT33 | CATGTAACCAC GACTACCTCAA (SEQ ID NO: 247) | CCATGGCTTG CAGCAATTT (SEQ ID NO: 248) | ACACTCTTTCC CTACACGACG CTCTTCCGATC TGTAACCACG ACTACCTCAAG ATATAA (SEQ ID NO: 249) | GTGACTGGAG TTCAGACGTGT GCTCTTCCGAT CTCACACAGA CGTACTGTTAA GGA (SEQ ID NO: 250) |
| OT34 | CTTAGAGGAA AGAGAACTGG GATTAT (SEQ ID NO: 251) | AGTGTGGCTG ATTATGGTGAT TA (SEQ ID NO: 252) | ACACTCTTTCC CTACACGACG CTCTTCCGATC TCCAAGAGTA GCCTAACCTTT ACAA (SEQ ID NO: 253) | GTGACTGGAG TTCAGACGTGT GCTCTTCCGAT CTCACGTAAAT TGCACCTGTC AC (SEQ ID NO: 254) |
| OT35 | TTTCTCTGCCA TTCTTCCTCTG (SEQ ID NO: 255) | GAATGAAGAC ACGAGGCATT TG (SEQ ID NO: 256) | ACACTCTTTCC CTACACGACG CTCTTCCGATC TTCTTAGCCCA | GTGACTGGAG TTCAGACGTGT GCTCTTCCGAT CTTCCAGAATG |
| | | | TGTTGCTTCC (SEQ ID NO: 257) | TACCTTGCACT TT (SEQ ID NO: 258) |
| OT36 | TGCTGTCTTTA GTTCCTTCATT (SEQ ID NO: 259) | TAACCCAGCA TCAGCTCTC (SEQ ID NO: 260) | ACACTCTTTCC CTACACGACG CTCTTCCGATC TTGCTGTCTTT AGTTCCTTCAT T (SEQ ID NO: 261) | GTGACTGGAG TTCAGACGTGT GCTCTTCCGAT CTTTAACCCAG CATCAGCTCTC (SEQ ID NO: 262) |
| OT37 | TTTCCAGAAGA GCCGACAAG (SEQ ID NO: 263) | CCAACAACCA CCACGACTAA (SEQ ID NO: 264) | ACACTCTTTCC CTACACGACG CTCTTCCGATC TGGGCCCTTC TGCTTTGAG (SEQ ID NO: 265) | GTGACTGGAG TTCAGACGTGT GCTCTTCCGAT CTAGTCTCCCA TGAAGGCTGT A (SEQ ID NO: 266) |
| OT38 | AAAGTACATAG AGGACGTGCA TAG (SEQ ID NO: 267) | AGTTCACCAC CACCACAAG (SEQ ID NO: 268) | ACACTCTTTCC CTACACGACG CTCTTCCGATC TTGTGCAAATA CTACGCCATTT C (SEQ ID NO: 269) | GTGACTGGAG TTCAGACGTGT GCTCTTCCGAT CTACAAGTTTG CACTTGCTTTC A (SEQ ID NO: 270) |
| OT39 | CACCTGGACC ACCAGAAA (SEQ ID NO: 271) | GCTGTTTGCAA ATGCCTCA (SEQ ID NO: 272) | ACACTCTTTCC CTACACGACG CTCTTCCGATC TCACCTGGAC CACCAGAAA (SEQ ID NO: 273) | GTGACTGGAG TTCAGACGTGT GCTCTTCCGAT CTACCCATCTC TGCAGACCTTA (SEQ ID NO: 274) |
| OT40 | CTGATTTCCTG AGTTTCTCCCT | AAGTGTGGGC TGTGCATAA | ACACTCTTTCC CTACACGACG | GTGACTGGAG TTCAGACGTGT |
| | AA (SEQ ID NO: 275) | (SEQ ID NO: 276) | CTCTTCCGATC TCTGTGAAGG GATTTCAAACT TTCC (SEQ ID NO: 277) | GCTCTTCCGAT CTCGATCAAG GCTAACGTCAT CA (SEQ ID NO: 278) |
| OT41 | CATCTCCTGCT GTGTCATCTT (SEQ ID NO: 279) | CCAGTCTCGG GTATGTCTTTA TT (SEQ ID NO: 280) | ACACTCTTTCC CTACACGACG CTCTTCCGATC TGACTGACTTC CATCTTCCTCA C (SEQ ID NO: 281) | GTGACTGGAG TTCAGACGTGT GCTCTTCCGAT CTCAGACTAAT ACATCCGGTC TCATC (SEQ ID NO: 282) |

Indels around a site 3 bp upstream of the PAM sequence were considered to be mutations resulting from Cas9 RNP activity.

### 7. RNA extraction and qPCR

Total RNA was isolated from NIH3T3 and ARPE-19 cells using an easy-spinTM Total RNA extraction Kit (iNtRON, Korea) according to the manufacturer's protocol. Then, 250 ng of RNA was reverse transcribed using SuperScript II (Enzynomics). Quantitative PCR (qPCR) was performed using SYBR Green (KAPA) with the following primers:
Mouse *Vegfa:* 5'-ACGTCAGAGAGCAACATCAC-3' (forward; SEQ ID NO: 283), 5'-CTGTCTTTCTTTGGTCTGCATTC-3' (reverse; SEQ ID NO: 284);
Mouse *Gapdh:* 5'-GCTGAGTATGTCGTGGAGTCTA-3' (forward; SEQ ID NO: 285), 5'-GTGGTTCACACCCATCACAA-3' (reverse; SEQ ID NO: 286);
Human *VEGFA-1:* 5'-CGAGTACATCTTCAAGCCATCC-3' (forward; SEQ ID NO: 287), 5'-GGTGAGGTTTGATCCGCATAAT-3' (reverse; SEQ ID NO: 288);
Human *VEGFA-2:* 5'-AGAAGGAGGAGGGCAGAAT-3' (forward; SEQ ID NO: 289), 5'-CACAGGATGGCTTGAAGATGTA-3' (reverse; SEQ ID NO: 290);
Human *GAPDH:* 5'-CAATGACCCCTTCATTGACC-3' (forward; SEQ ID NO: 291), 5'-TTGATTTTGGAGGGATCTCG-3' (reverse; SEQ ID NO: 292).

### 8. VEGFA ELISA using confluent ARPE-19 cells

For human VEGFA ELISA, Vegfa-specific Cas9 RNP-treated confluent ARPE-19 cells were incubated in a serum-free medium for 16 hours after which serum-free supernatants were collected from the cell culture. Secreted VEGFA protein levels were measured using a human VEGF Quantikine ELISA Kit (DVE00, R & D systems) according to the manufacturer's instructions.

### 9. In vitro cleavage of genomic DNA and Digenome sequencing

Genomic DNA was isolated from ARPE-19 cells (ATCC) using a DNeasy Tissue Kit (Qiagen). In vitro cleavage of genomic DNA for Digenome sequencing was performed as described below. In brief, genomic DNA (20 µg) was incubated with Cas9 protein (16.7 µg) and sgRNA (12.5 µg) in reaction buffer (100 mM NaCl, 50 mM Tris-HCI, 10 mM MgCl₂, 100 µg/mL BAS, pH 7.9) for 3 hours at 37°C to induce the cleavage of genomic DNA by Cas9. Cleaved genomic DNA was treated with RNase A (50 µg/mL, Sigma Aldrich) for 30 min at 37°C and purified with a DNeasy Tissue Kit (Qiagen). Whole-genome and Digenome sequencing were performed as described previously (Kim, D., Kim, S., Kim, S., Park, J. & Kim, J. S. Genome-wide target specificities of CRISPR-Cas9 nucleases revealed by multiplex Digenome-seq. Genome research 26, 406-415 (2016)).

### 10. Preparation of Animals Administered with RNP by Subretinal Injection

The care, use, and treatment of all animals in this study were in strict agreement with the guidelines established by the Seoul National University Institutional Animal Care and Use Committee and "the ARVO Statement for the Use of Animals in Ophthalmic and Vision Research". Adult (6 weeks old) male SPF C57BL/6J mice were used in the study. Mice were maintained under a 12 hours dark-light cycle.

Subretinal injection of RNP was performed on the prepared mice as follows. First, RNPs composed of Cas9 protein (8 µg), sgRNA (4.5 µg), and Lipofectamine 2000 (20% v/v) were mixed in 2 µL or 3 µL of injection volume. RNPs (2 or 3 µL) were injected into the subretinal space using a Nanofil syringe with a 33G blunt needle (World Precision Instruments, Inc.) under an operating microscope (Leica Microsystems, Ltd.). Subjects with retinal hemorrhage were excluded from the study.

### 11. Construction of Laser-Induced Choroidal Neovascularization (CNV) Animal Models

Mice were anesthetized by intraperitoneally injecting at a dose of 2.25 mg/kg (body weight) a mixture containing tiletamine and zolazepam at a weight ratio of 1:1. Pupils were dilated with an eye drop containing phenylephrine (0.5% (w/v)) and tropicamide (0.5% (w/v)). Laser photocoagulation was performed using an indirect head set delivery system (Iridex) and laser system (Ilooda). The laser wavelength was 810 nm. Laser parameters were as follows: spot size: 200 µm; power: 1W; and exposure time: 100 ms. Laser bum was induced at the 12 (right eye) or 6 (left eye) o'clock positions around the optic disc with a modification. Only bums that produced a bubble without vitreous hemorrhage were included in the experiments. Subretinal RNP injections were performed in the quadrant of laser burn. Cas9 RNPs (sgRosa26 (containing a Rosa26 targeting sgRNA) or sgVegfa (containing a Vegfa targeting sgRNA)) were randomly allocated to the left or right eye in each mouse. Subretinal injection of Cas9 RNPs produced a bleb. It was confirmed that the bleb overlapped with the laser-burn site. Subjects in which the bleb overlapped the laser-burn site were used in further studies. Seven days after laser treatment, the eyes were fixed in 4% PFA for 1 hour at room temperature. RPE (retinal pigment epithelium) complexes (RPE/choroid/sclera) were treated ovemight at 4°C with isolectin-B4 (Thermo Fisher Scientific, catalog no. I21413, 1:100) for immunostaining. The stained RPE complexes were flat-mounted and viewed with a fluorescence microscope (Eclipse 90i, Nikon) at a magnification of x40. The CNV area was measured using ImageJ software (1.47v, NIH) by blind observers.

### 12. Immunosfluorescent Staining and Imaging

The number of RPE cells in the RPE complex was calculated by counting DAPI-stained nuclei in paraffin embedded cross-section samples (4 µm) in a high power field area (100 µm × 100 µm, n = 8). Cross-section samples obtained at day 7 post-injection (n = 4) were immunostained with an anti-opsin antibody (Millipore, AB5405, 1:1000) and an Alexa Fluor 488 antibody (Thermo Fisher Scientific, 1:500). The opsin positive area was measured using ImageJ software (1.47v, NIH) by blind observers. The intracellular distribution of the Cy3-Cas9 protein in the RPE flat-mounts was imaged using a confocal microscope (LSM 710, Carl Zeiss). The scanning parameters were as follows: scaling (x = 0.042 µm/pixel, y = 0.042 µm/pixel, z = 0.603 µm/pixel), dimensions (x = 1024, y = 1024, z = 12, channels: 2, 8-bit), and zoom (5.0) with objective C-Apochromat 40×/1.20W Korr M27. ZEN 2 software was used to process the images.

### 13. Genomic DNA Extraction from CNV Area and RPE Complex

Genomic DNA was isolated from RPE complexed at day 3 after RNP injection, and measured for CNV area. At day 7 after RNP injection, genomic DNA was isolated from CNV samples. Genomic DNA isolation was performed using a NucleoSpin Tissue Kit (Macherey-Nagel). In order to evaluate RNP efficacy, each RPE complex was divided into quadrants and the RNP-injected quadrant was treated to isolate genomic DNA. To assess indel frequencies in CNV areas of RPE complexes, RPE flat-mounts were imaged and washed with PBS. Genomic DNA was isolated from the following regions: (i) a quadrant including an RNP-injected area of CNV (representative of injected areas); and (ii) an opposite quadrant (representative of non-injected areas).

### 14. Mouse VEGF-A ELISA

For mouse VEGF-A ELISA, a total of 30 laser burns were induced in the eye, followed by injecting RNPs (3 µL) into the subretinal space. At day 3 post-injection, whole RPE complexes were isolated from the retina and frozen for further analysis. Cells were lysed with RIPA buffer (50 mM Tris-HCl(pH 8.0), 150 mM NaCl, 1% Igepal CA-630, 0.5% Na.deoxycholate, 0.1% SDS), and VEGFA levels were measured using a mouse VEGF Quantikine ELISA Kit (MMV00, R&D systems) according to the manufacturer's instructions.

### 15. Western blotting

To analyze RNP levels over time after in vivo RNP delivery, Westem blotting of RPE complexes, obtained at 1 and 3 days post-injection, was performed. Samples, each containing an equal amount of protein (20 µg), were analyzed; Cas9 and beta-actin were detected with an anti-HA high affinity antibody (Roche, 3F10, 1:1000) and an anti-beta-actin antibody (Sigma Aldrich, A2066, 1:1000), respectively. ImageQuant LAS4000 (GE healthcare) was used for digital imaging.

### 16. Statistics

Data were analyzed with SPSS software version 18.0 (SPSS, Inc.). P-values were determined by an unpaired, two-sided Student's t-tests or one-way ANOVA and Tukey post-hoc tests (for multiple groups). Data are expressed as mean with s.e.m (standard error of the mean).

### EXAMPLE 1: Target Mutation of Vegfa/VEGFA Gene through Cas9 Ribonucleoproteins (RNPs)

A test was made of four single-chain guide RNAs (sgRNAs) (labeled as Vegfa-1, 2, 3, and 4) targeting target sites in exons 3 and 4 which encode binding sites for VEGF receptors 1 and 2, respectively, in the mouse NIH3T3 cell line and the human RPE cell line (ARPE-19). The four sgRNAs (Vegfa-1, 2, 3, and 4) were constructed with reference to Reference Example 1.

The targeting sequences of sgRNA for CRISPR-Cas9 target sequences in the *VEGFA*/*Vefga* gene and numbers of homologous sites in the human and mouse genomes are summarized in Table 6, below:

**Table 6**

| **Target** | **sgRNA with PAM (5' to 3')** | **Position** | **Direction** | **Number of mismatches at homologous sites*** | | |
|---|---|---|---|---|---|---|
| | | | | 0 | 1 | 2 |
| *Vegfa*-1 human mouse | CTCCTGGAAGATGTCCACCAG GG (SEQ ID NO: 293) | Exon 3 | - | 1 | 0 | 1 |
| | | | | 1 | 0 | 1 |
| *Vegfa*-2 human | AGCTCATCTCTCCTATGTGCT | Exon 4 | - | 1 | 0 | 1 |
| mouse | GG (SEQ ID NO: 294) | | | 1 | 0 | 3 |
| *Vegfa*-3 human mouse | GACCCTGGTGGACATCTTCCA GG (SEQ ID NO: 295) | Exon 3 | + | 1 | 0 | 0 |
| | | | | 1 | 0 | 1 |
| *Vegfa*-4 human mouse | ACTCCTGGAAGATGTCCACCA GG (SEQ ID NO: 296) | Exon 3 | - | 1 | 0 | 1 |
| | | | | 1 | 0 | 0 |

| | | | | | | |
|---|---|---|---|---|---|---|
| (* Determined using Cas-OFFinder (http://www.rgenome.net/cas-offinder/); underlined: PAM sequence) | | | | | | |

The sgRNA-containing Vegfa-specific Cas9 RNP prepared above were transfected into mouse NIH3T3 and human ARPE-19 cells and used in the following experiments. The delivery of RNP into cells, as described in Reference Example 3, was carried out with the aid of a plasmid that was introduced into cells and allowed nucleic acid molecules to express sgRNA and Cas9 protein in the cells (expressed as plasmid in figures) or in such a manner that a complex (or mixture) of the sgRNA and the recombinant Cas9 protein was delivered into cells using cationic lipid (Lipofectamine) (expressed as RNP in figures).

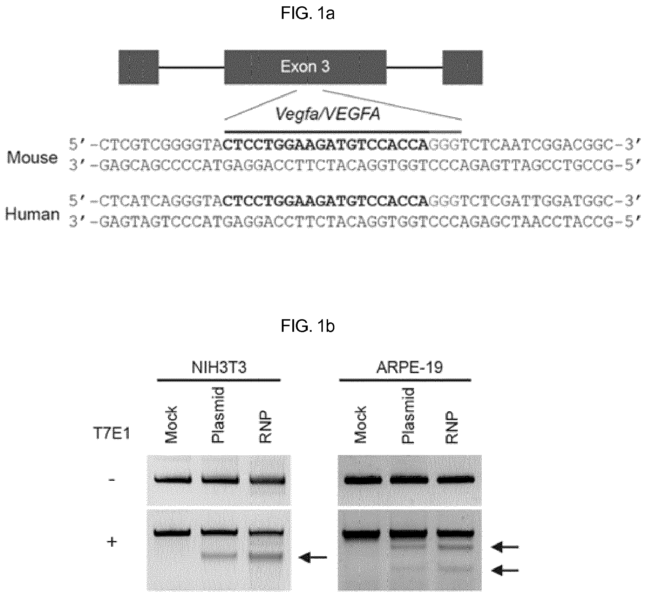

The target sequence in the *VegfalVEGFA* locus of mouse NIH3T3 and human ARPE-19 cells are depicted in FIG. 1a (PAM sequence in red; sgRNA target sequence in blue).

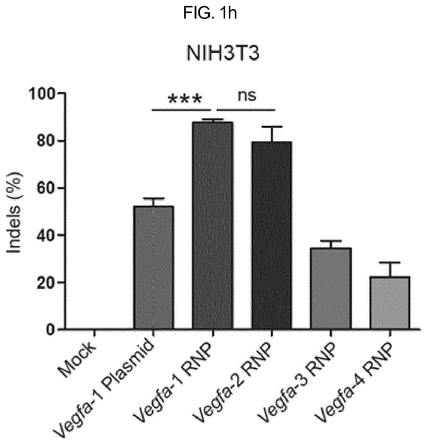

Mutation frequencies induced by the delivery of either Vegfa-specific Cas9 RNPs containing the four sgRNAs (Vegfa-1 sgRNA, Vegfa-2 sgRNA, Vegfa-3 sgRNA, and Vegfa-4 sgRNA) or plasmids carrying encoding sequences thereof were measured using deep sequencing (see Reference Example 2) at day 2 post-transfection. The results are shown in FIG. 1h (Error bars indicate s.e.m. (n=3), One-way ANOVA and Tukey post-hoc tests, *** *P <* 0.001).

In addition, mutations induced by the delivery of either Vegfa-specific Cas9 RNP containing Vegfa-1 sgRNA or a plasmid carrying an encoding sequence thereof in NIH3T3 and ARPE-19 cells were detected by the T7 endonuclease I (T7E1) assay and are shown in FIG. 1b. In FIG. 1b, arrows indicate the expected positions of DNA bands cleaved by T7E1.

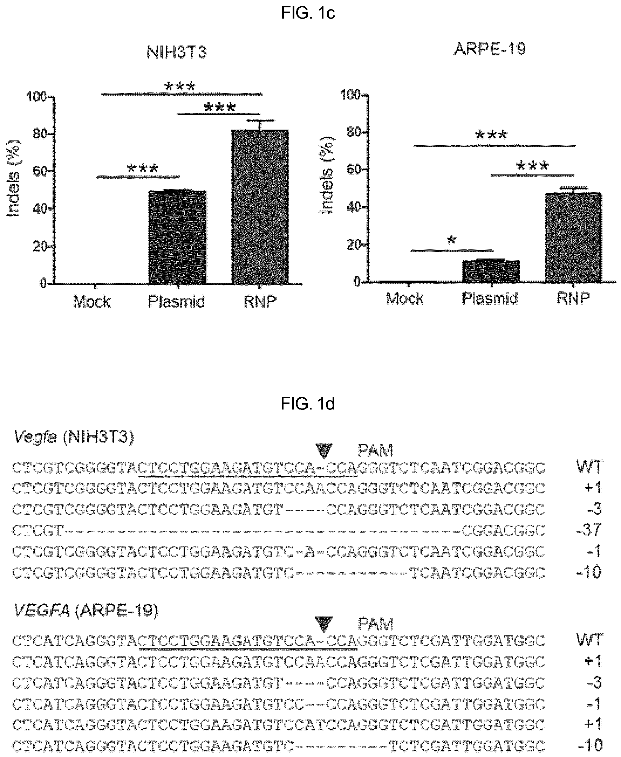

Indel frequencies induced by the delivery of either Vegfa-specific Cas9 RNP containing Vegfa-1 sgRNA or a plasmid carrying an encoding sequence thereof in NIH3T3 and ARPE-19 cells were measured using targeted deep sequencing (see Reference Example 6) at day 2 post-transfection. The results are shown in FIG. 1c (error bar: s.e.m (n=3); One-way ANOVA and Tukey post-hoc tests, * *P* < 0.05, ** *P <* 0.01, *** *P* < 0.001).

Representative mutant DNA sequences induced by Vegfa-specific Cas9 RNP (containing Vegfa-1 sgRNA) at the *Vegfa*/*VEGFA* locus in NIH3T3 and ARPE-19 cells are shown in FIG. 1d (underlined: target sequence targeted by sgRNA, blue: inserted nucleotides, -: deleted region, WT: wild type; triangle: cleavage position; column on the right: number of inserted or deleted nucleotides).

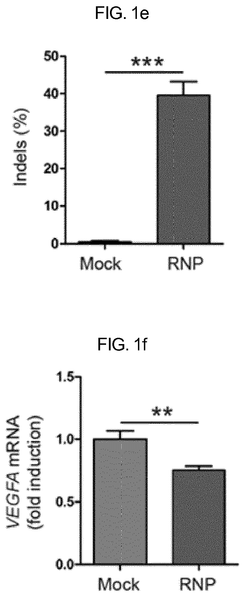

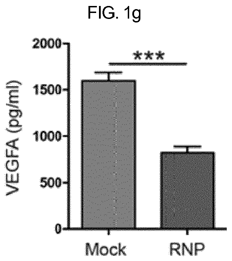

Mutation (indel) frequencies induced by the delivery of Vegfa-specific Cas9 RNP containing Vegfa-1 sgRNA in confluent ARPE-19 cells were detected by targeted deep sequencing at 64 hours post-transfection, and the results are given in FIG. 1e. Relative VEGFA mRNA levels measured in the cells by quantitative PCR (qPCR) are depicted in FIG. 1f, and VEGFA protein levels measured in the cells by VEGFA ELISA as in Reference Example 8 are shown in FIG. 1g (Error bar: s.e.m. (n = 5), Student's t-test, ** *P <* 0.01, **** P <* 0.001).

As shown in FIG. 1h, the highest indel efficiency was obtained in NIH3T3 cells when Vegfa-1 sgRNA among the four sgRNAs was complexed with Cas9 and delivered in the RNP form into the cells. In addition, FIGS. 1b and 1c show that Vegfa-1 sgRNA with the highest indel efficiency induced mutations in NIH3T3 and ARPE-19 cells and when delivered in an RNP form, was observed to induce small insertions and deletions (indels) at the target site with a frequency of 82±5 % (NIH3T3 cells) or 57±3 % (ARPE-19 cells). The RNP delivery of sgRNA and Cas9 allowed higher indel efficiency than plasmid transfection (FIG. 1c).

As shown in FIG 1e, indels were detected at a frequency of 40 ± 8% in ARPE-19 cells treated with Vegfa-specific Cas9 RNP, and FIGS. 1f and 1g show that Vegfa-specific Cas9 RNP reduced the VEGFA mRNA level by 24 ± 4% and the VEGFA protein level by 52 ± 9% in confluent ARPE-19 cells under post-mitotic conditions.

### EXAMPLE 2: In vitro and In vivo Delivery of Cy3-labeled Cas9 RNP

To monitor the localization of Cas9 RNPs in vitro and in vivo, Cy3-conjugated Cas9 protein (Reference Example 4). Thus, Cy3-Cas9 combined with or without the *Vegfa-1* sgRNA was mixed with cationic lipids and delivered into NIH3T3 cells. Alternatively, the Vegfa-specific, Cy3-labeled or -unlabeled Cas9 RNP was delivered into an adult mouse eye via subretinal injection for the following experiments.

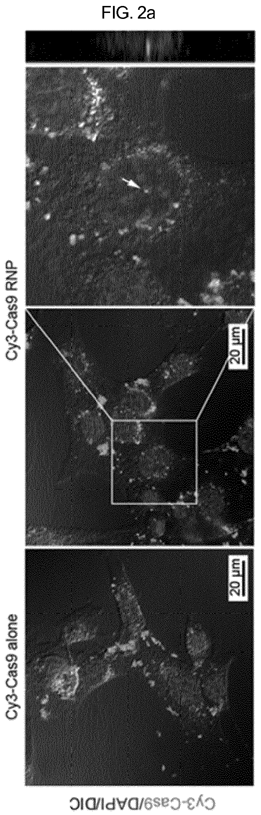

NIH3T3 cells were transfected with Cy3-labeled Cas9 RNP (a complex of Cy3-labeled Cas9 and Vegfa-1 sgRNA) or Cy3-labeled Cas9 alone (as a control) and observed under a confocal microscope at 24 hours post-transfection to visualize Cy3 signals in the cells (see Reference Example 4). The images thus obtained are given FIG. 2a. In FIG. 2a, the white arrow indicates nuclear co-localization of Cy3 dye. The z-axis image on the right shows that Cy3-Cas9 is localized inside the nucleus.

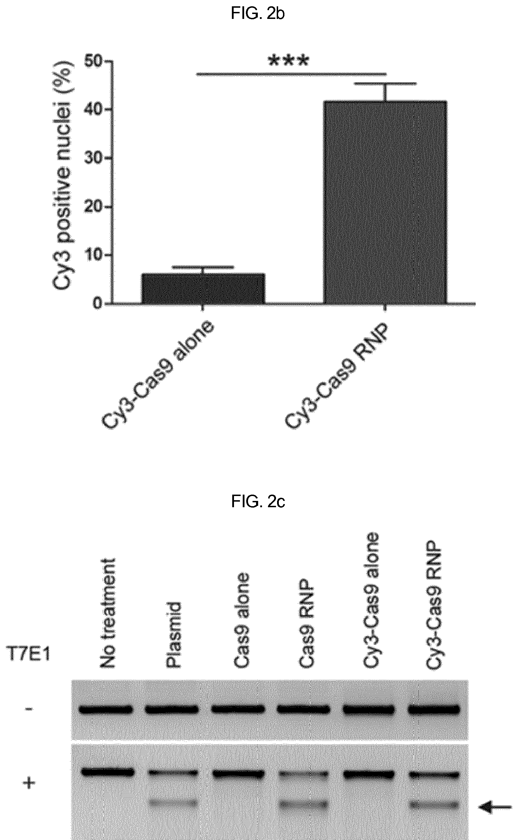

In addition, measurement was made of the proportion of Cy3 positive nuclei in total DAPI positive nuclei (100*[number of Cy3-positive nuclei]/[total number of DAPI-positive nuclei]) at 24 hours post-transfection, and the results are depicted in FIG. 2b (Error bars indicate SEM *(n* = 3). Student's *t*-test: *** *P <* 0.001).

Mutations mediated by Vegfa-specific Cas9 RNP containing the Vegfa-1 sgRNA in NIH3T3 cells were detected by the T7 endonuclease 1 (T7E1) assay (see Reference Example 5) and are shown in FIG. 2c. In FIG. 2c, the arrow indicates the expected position of DNA bands cleaved by T7E1.

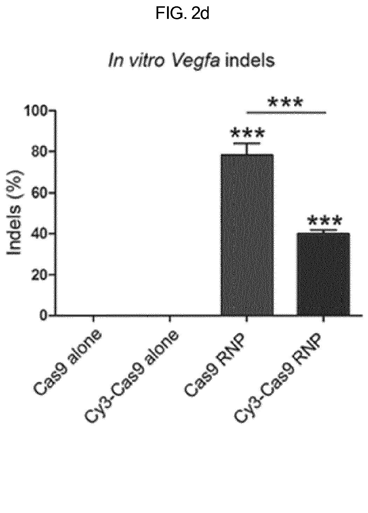

Mutation frequencies driven by the Vegfa-specific Cas9 RNP containing the Vegfa-1 sgRNA in NIH3T3 cells were measured using targeted deep sequencing (see Reference Example 6) at 24 hours post-transfection and are shown in FIG. 2d (error bar: s.e.m (n=3); One-way ANOVA and Tukey post-hoc tests, *** *P* < 0.001).

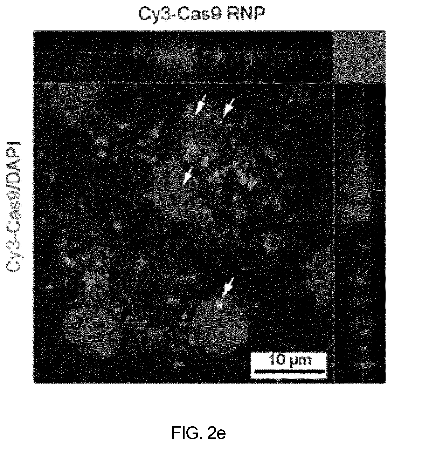

Representative RPE flat-mount at day 3 after the injection of Cy3-labeled Cas9 RNP into mouse eye was observed under a fluorescence microscope (see Reference Examples 11 and 12) and the results are given in FIG. 2e. In this figure, white arrows indicate nuclear colocalization of Cy3 dye.

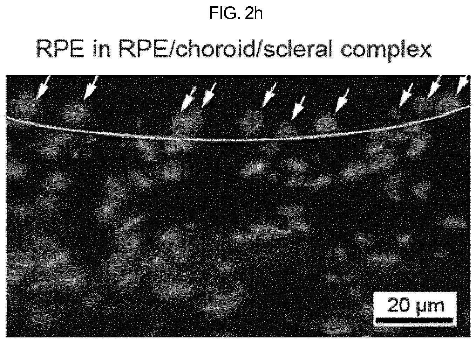

Distribution of retinal pigment epithelium (RPE) in the RPE/choroid/scleral complex was observed under a fluorescence microscope (see Reference Examples 11 and 12), and the results are shown in FIG. 2h. FIG. 2h depicts a representative cross-section of the RPE/choroid/scleral complex. DAPI-positive RPE cells and other cells were counted in a high-power field area (100 µm x 100 µm). In FIG. 2h, the yellow line indicates a boundary between RPE and choroid and white arrows indicate RPE nuclei in the RPE/choroid/scleral complex (10.5±2.8%, n=8).

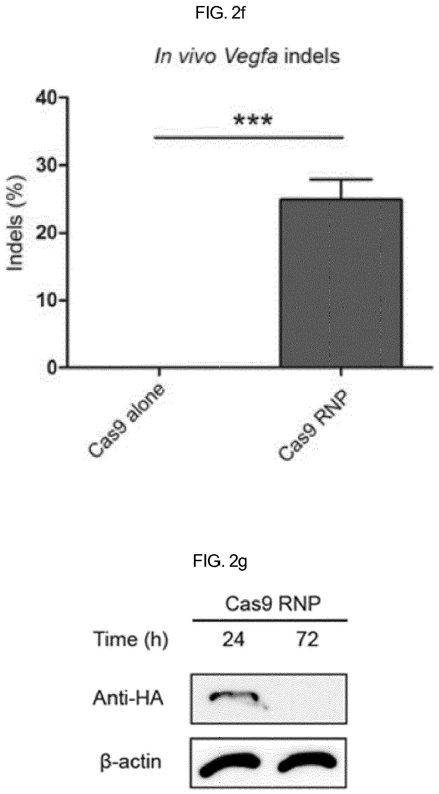

Indel frequencies induced in vivo were determined using genomic DNA isolated from the retinal pigment epithelium (RPE)/choroid/scleral complexes, with reference to Reference Example 13. Indels were analyzed by deep sequencing (see Reference Example 6) at day 3 post-injection. The results are depicted in FIG. 2f (Error bars are s.e.m. (n=6), Student's t-test, ** *P* < 0.01).

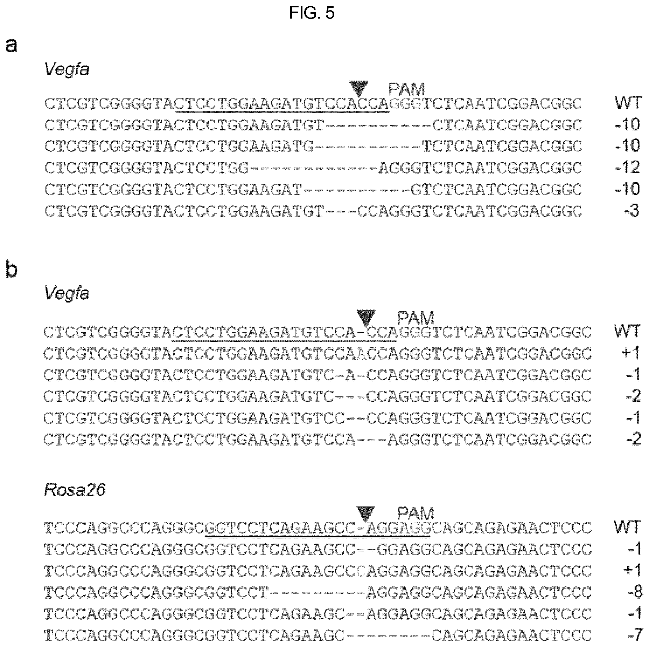

Mutant DNA sequences induced by Vegfa-specific Cas9 RNPs (containing Vegfa-1 sgRNA) *in vivo* are depicted in FIG. 5: (a) Representative mutant DNA sequences induced by the Vegfa-specific Cas9 RNP in RPE at day 3 post-injection; and (b) Mutant DNA sequences in RPE containing laser-induced choroidal neovascularization (CNV) at day 7 post-injection. The PAM sequence is shown in red. WT denotes wild-type. The column on the right indicates the number and frequency of inserted or deleted nucleotides.

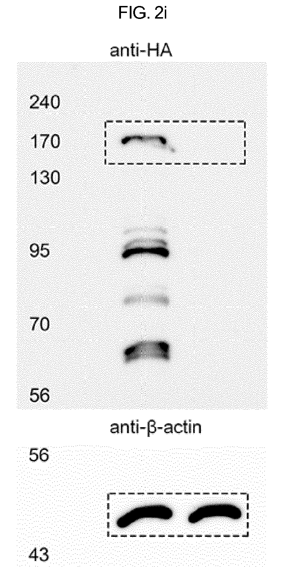

Western blot analysis was performed to measure the level of Cas9 protein in the RPE/choroid/scleral complex 24 and 72 hours after injection (n = 4), and the results are shown in FIGS. 2g and 2i.

FIGS. 2a to 2d show in vitro results. Cy3-Cas9 RNP was detected in many nuclei, as shown in FIGS. 2a and 2b, and was observed to induce indels at the target site, as shown in FIGS. 2c and 2d.

The proportion of Cy3 positive nuclei (42 ± 6%) (FIG. 2b) upon treatment with Cy3-Cas9 RNP was almost equal to the frequency of indels (40 ± 3%) (FIG. 2d) at the target site, suggesting that target sites were almost completely cleaved in cells by nucleus-localized Cas9 and that the rate-limiting factor in genome editing was nuclear localization of Cas9. When delivered alone without sgRNA, Cy3-Cas9 was rarely detected in nuclei and did not induce indels (FIGS. 2a and 2d). Cas9 is a positively charged protein with a pl value of 9.12 and cannot form a complex with cationic lipids in the absence of negatively charged sgRNAs. However, the Cy3-Cas9 RNP was less active than the unlabeled Cas9 RNP, which induced target-specific mutations at a frequency of 80% (FIG. 2d).

FIGS. 2e to 2i show in vivo results. Cy3 dye was observed in the nuclei of the RPE in vivo 3 days after the injection of Cy3-Cas9 RNP (in vivo, FIG. 2e). Because RPE is a major target of the RNP delivery by retinal injection, RPE alone allows for the ideal analysis of mutation frequencies. Actually, however, it is not easy to classify RPE for use in targeted deep sequencing from the RPE/choroid/scleral complex. Instead, DAPI-positive nuclei were counted to calculate the proportion of RPE, which accounted for 11±3% of the RPE/choroid/scleral complex (FIG. 2h).

Notably, the subretinal injection of the Cy3-unlabeled Cas9 RNP gave rise to indels with a frequency of 16±2% at day 3 post-injection, with the consequent editing in most target sites of the Vegfa gene in RPE in vivo (n=6, FIGS. 2f and 5a). Through Western blot analysis, it was also found that the Cas9 protein was degraded completely at day 3 post-injection (FIGS. 2g and 2i), indicating that Cas9 was rapidly turned over in vivo.

### EXAMPLE 3: Assay for Effect of Retinal Injection of Vegfa-Targeting Cas9 RNP on Age-Related Macular Degeneration (AMD) and Laser-induced Choroidal Neovascularization (CNV)

In order to investigate whether the Cas9 RNP could be used for the treatment of CNV in AMD mouse models, mice with laser-induced CNV were treated by subretinal injection of the Vegfa-specific Cas9 RNP (containing Vegfa-1 sgRNA) or Rosa26-specific Cas9 RNP (Rosa26-RNP; containing Rosa26 sgRNA). Since retinal injection itself increases the size of CNV, the Rosa26-RNP was used as a negative control.

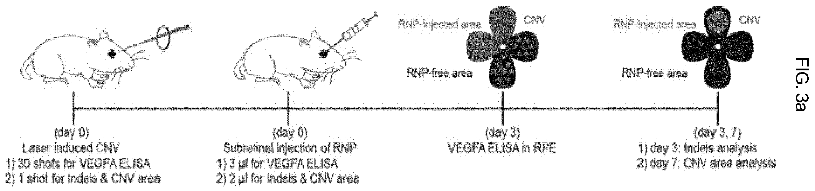

Mice with laser-induced CNV were administered with the preassembled Vegfa-specific Cas9 RNP by retinal injection. After the retinal pigment epithelium (RPE) complex in the eye was flat-mounted, the CNV area was analyzed at day 7 post-injection. Genomic DNA isolated from the Cas9 RNP-injected area or from the opposite non-injected area (RNP-free area) was analyzed by deep sequencing. Vegfa ELISA was performed at day 3 post-injection. This procedure is schematically shown in FIG. 3a.

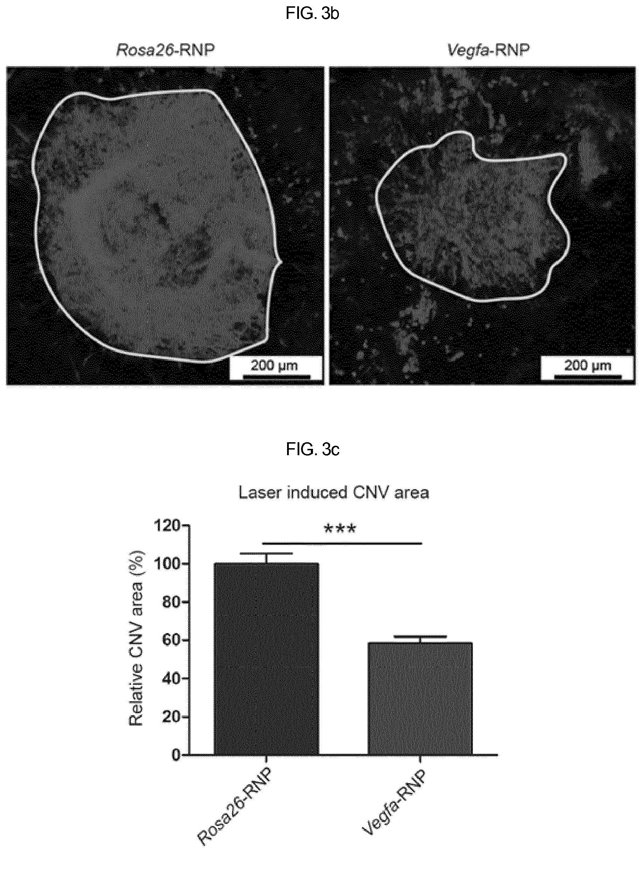

Laser-induced CNV stained with isolectin B4 (IB4) in C57BL/6J mice injected with the Rosa26-specific Cas9 RNP (as a control) or the Vegfa-RNP at day 7 post-injection was visualized (see Reference Example 11). Representative images are shown in FIG. 3b. The yellow line demarcates the area of CNV.

At day 3 post-injection, a therapeutic effect was evaluated by assessing the CNV area. CNV areas were measured in C57BL/6J mice injected with the Vegfa-specific Cas9 RNP with reference to Reference Example 11 and are depicted as relative values (%) to the CNV area (100 %) of the control injected with the Rosa26-specific Cas9 RNP in FIG. 3c (Error bars indicate s.e.m. (n=15), Student's t-test, *** *P <* 0.001).

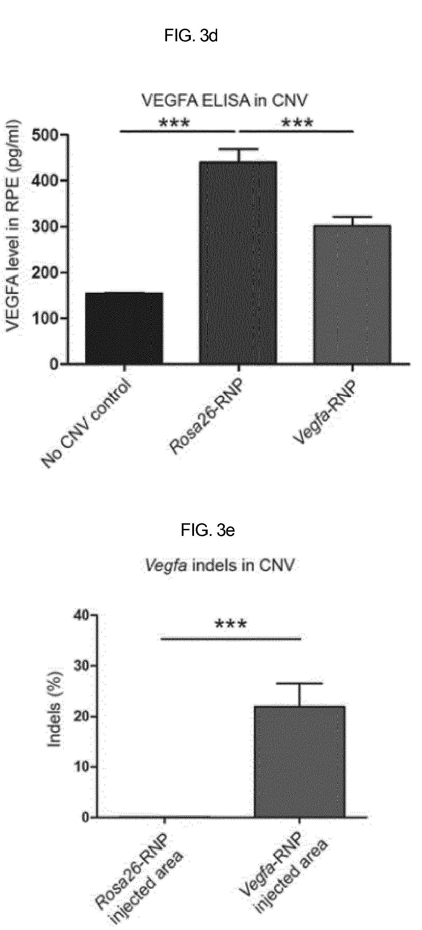

Vegfa levels (pg/ml) in CNV areas were measured by ELISA (see Reference Example 14), and the results are shown in FIG. 3d (Error bars indicate s.e.m. (n=10), Student's t-test, ** *P* < 0.01).

Indel frequencies (%) at the Vegfa target site in the RPE complex are shown in FIG. 3e (Error bars indicate s.e.m. (n=7), One-way ANOVA and Tukey post-hoc tests, *** P < 0.001).

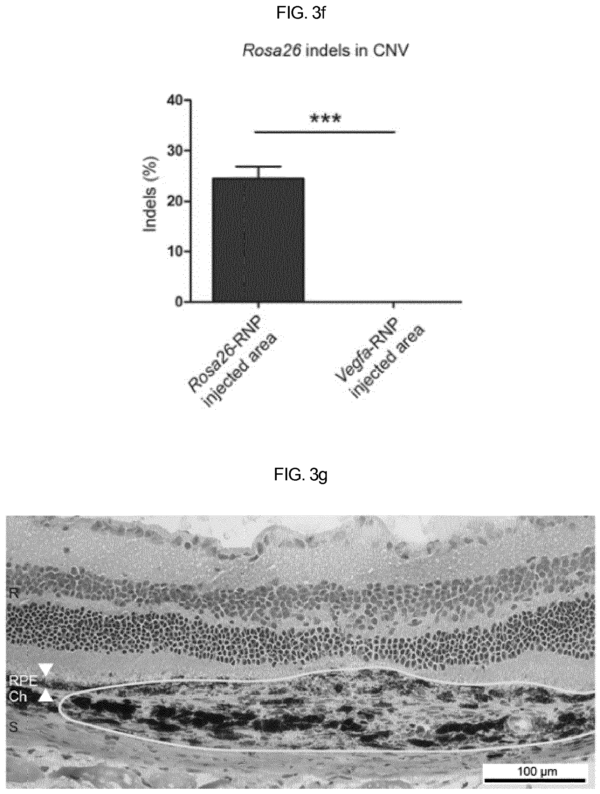

Indel frequencies (%) at the *Rosa26* target site in the RPE complex are shown in FIG. 3f (Error bars indicate s.e.m. (n=7), Student's t-test, * *P* < 0.05).

The laser-induced CNV structure in a cross-section sample was visualized by hematoxylin & eosin staining and is shown in FIG. 3g. In FIG. 3g, the yellow line indicates a CNV boundary and white triangles indicate a retinal pigment epithelium (RPE) layer in the RPE/choroid/scleral complex. Most RPE cells were observed to be injured in the CNV area (Ch: choroid, R: retina, S: sclera).

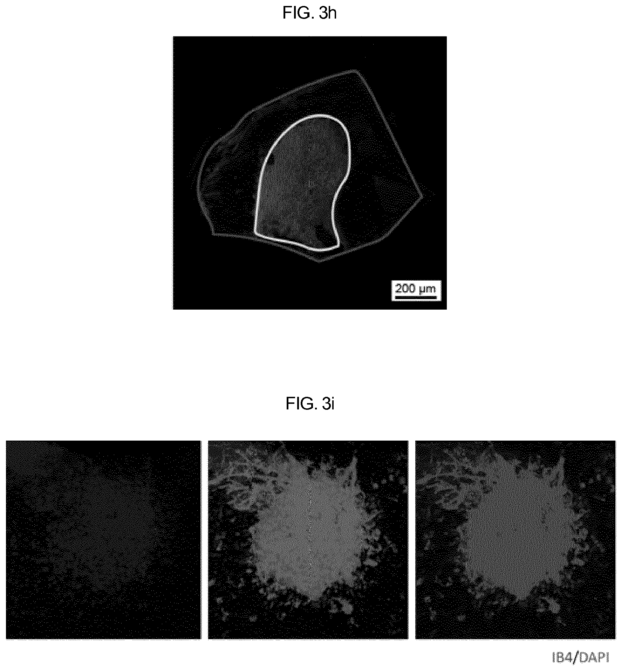

A representative CNV sample for use in mutation analysis through targeted deep sequencing is shown in FIG. 3h. In FIG. 3h, the red line indicates a boundary of the RPE/choroid/scleral complex for mutation analysis. RPE cells predominantly existed outside the CNV area limited by the yellow line.

Laser-induced CNV at day 7 after laser treatment is depicted in FIG. 3i. Epithelial cells co-stained with IB4 marker and DAPI were collected into the CNV areal (middle).

As shown in FIGS. 3b and 3c, a significant reduction of CNV area was found in the Vegfa-RNP-injected mice, compared to the Rosa26-RNP-injected mice (58±4% relative to Rosa26-RNP-injected mice, n = 15, P <0.001, Student's t-test).

In addition, as shown in FIG. 3d, the injection of Vegfa-specific Cas9 RNP effectively reduced the concentration of the Vegfa protein (300±20pg/ml, n=10)) in the CNV area, compared to the injection of Rosa26-RNP (440±30pg/ml, n=10).

As understood from FIGS. 3e and 3f, indel frequencies in RNP target sites in the Vegfa-specific Cas9 RNP-treated CNV and the Rosa26-RNP-treated CNV (see FIG. 5b) were detected to be 3.5±0.3% (Vegfa indel) and 3.3±1.0% (Rosa26 indel), respectively, whereas indels were not detected in the negative control at all. This result suggests that subretinal injection of the Vegfa-specific Cas9 RNP can lead to local treatment even in the eye.

FIG. 3g shows that most RPE cells in CNV were killed by laser treatment. Gene editing by Cas9 RNP was not effected in dead cells, but only in viable RPE cells (FIG. 3h). As a result, lower indel frequencies were detected in viable RPE cells than in cells in CNV-free areas. At day 3 after laser treatment, epithelial cells gathered in the CNV area to form new vessels (FIG. 3i). Indel frequencies further decreased I the CNV area as the cells were not be gene edited. These results suggest that that targeted inactivation of Vegfa in the eye using Cas9 RNPs enables therapeutic genome surgery for the local treatment of AMD.

A critical issue in therapeutic genome therapy is the target specificity of CRISPR-Cas9. It was examined whether the Vegfa-specific Cas9 RNP used in this experiment caused any off-target mutations in the mouse eye or in human cells. First, 20 potential off-target sites in the mouse genome that are the most homologous to the target sequence of Vegfa-sgRNA of the Cas9 RNP were identified using Cas-OFFinder and are summarized in Table 7, below.

**Table 7**

| Potential off-target sites of the Vegfa-1 sgRNA (with PAM) in the mouse genome | | | | | | |
|---|---|---|---|---|---|---|
| **No.** | **Gene** | | **Sequence** | **Chromosome** | **Position** | **Direction** |
| On | *Vegfa* | Exon | CTCCTGGAAGATGTCCACC AGGG (SEQ ID NO: 293) | chr17 | 46025487 | - |
| OT1 | Intergenic region | - | CTCCTGGAAGATTTTCACC AGGG (SEQ ID NO: 297) | chr2 | 1230234 49 | - |
| OT2 | *Arhgef7* | Intron | CTCCTGGAAGATCTCCAGG AAGG (SEQ ID NO: 298) | chr8 | 11735358 | - |
| OT3 | *Gsc* | Exon | CTCCTGGAAGAGGTTCTCC AGGG (SEQ ID NO: 299) | chr12 | 104472064 | + |
| OT4 | *Ptpm2* | Exon | CTCTTGGCAGATGTCCACA AGGG (SEQ ID NO: 300) | chr12 | 116842612 | + |
| OT5 | *Gpr139* | Intron | CTCCTGGAAGCTGCCCATC ATGG (SEQ ID NO: 301) | chr7 | 119178456 | - |
| OT6 | *Kank4* | Intron | CTCCTGGAAAATGCCCACC CTGG (SEQ ID NO: 302) | chr4 | 98816689 | + |
| OT7 | *Stk32b* | Intron | CTCCTGGAAGATGTGGGC CATGG (SEQ ID NO: 303) | chr5 | 37675822 | - |
| OT8 | *Ptpn11* | Intron | CTCCTGAAAGCTGACCAC CACGG (SEQ ID NO: 304) | chr5 | 121146230 | + |
| OT9 | *Acad12* | Intro | CACATGGAGGATGTCCAC | chr5 | 1216062 | + |
| | | n | CATGG (SEQ ID NO: 305) | | 39 | |
| OT10 | Intergenic region | - | CTCCTGGAAGCTGTTGACC AGGG (SEQ ID NO: 306) | chr5 | 138824186 | + |
| OT11 | Intergenic region | - | CTCCTGGAAGAGGACAAC CAAGG (SEQ ID NO: 307) | chr13 | 44510080 | - |
| OT1 2 | *Fibp* | Exon | CTGCTGGATGTTGTCCACC AGGG (SEQ ID NO: 308) | chr19 | 5462580 | - |
| OT1 3 | Intergenic region | - | CTCCTGGAAGTTGTCCTCC TTGG (SEQ ID NO: 309) | chr15 | 8736050 2 | - |
| OT1 4 | Intergenic region | - | CCCCTGGAAGATTTCCATC AAGG (SEQ ID NO: 310) | chr17 | 3779341 1 | + |
| OT1 5 | Intergenic region | - | CTCTTGGCAGCTGTCCACC ATGG (SEQ ID NO: 311) | chr10 | 2436558 5 | - |
| OT1 6 | Intergenic region | - | CTCCAAGAAGATGTCCTCC ATGG (SEQ ID NO: 312) | chr10 | 5586909 8 | - |
| OT1 7 | *Fam180a* | Exon | CTCCTGGAAGATGTCCTGG AAGG (SEQ ID NO: 313) | chr6 | 3532590 1 | - |
| OT1 8 | *Rasgrf1* | Exo n | CTCCTGGTAGATGTTCAGC ATGG (SEQ ID NO: 314) | chr9 | 8997037 6 | - |
| OT1 9 | *Abca13* | Intro n | GTCCTGGAAGCTGTCCAC AAAGG (SEQ ID NO: 315) | chr11 | 9509771 | + |
| OT2 0 | *C130046K22 Rik* | Intro n | CTCAGTGAAGATGTCCACC AAGG (SEQ ID NO: 316) | chr11 | 1037133 36 | + |

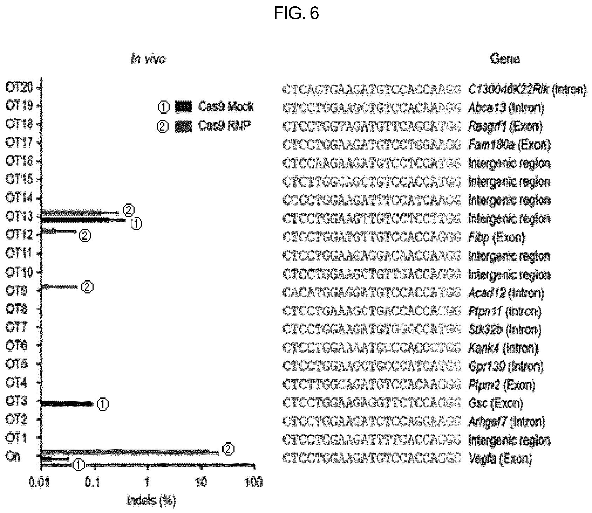

Genomic DNA isolated from the CNV-free RPC complex of the Cas9 RNP-treated mouse eye was subjected to targeted deep sequencing to indel frequencies (%) in the 20 potential off-target sites. The results are depicted in FIG. 6 (Error bars indicate s.e.m. (n=3 for Cas9 Mock, n=5 for Cas9 RNP, respectively). Mismatched nucleotides and PAM sequences are shown in red and blue, respectively. At the 20 potential off-target sites treated with the Cas9 RNP, no Cas9-induced indels were detected with a frequency greater than 0.1 %, demonstrating that no off-target mutations were induced above sequencing error rates (sequencing error rate; average 0.1%). That is, the Vegfa-specific Cas9 RNP used in the present disclosure was found to exhibit no off-target effects in RPE.

### EXAMPLE 4: Assay for Genome-Wide Target Specificity of Vegfa-Specific Cas9 RNP in Human Genome

Genome-wide target specificity of the Vegfa-specific Cas9 RNP in the human genome was revealed by Digenome-seq (see Reference Examples 6 and 9).

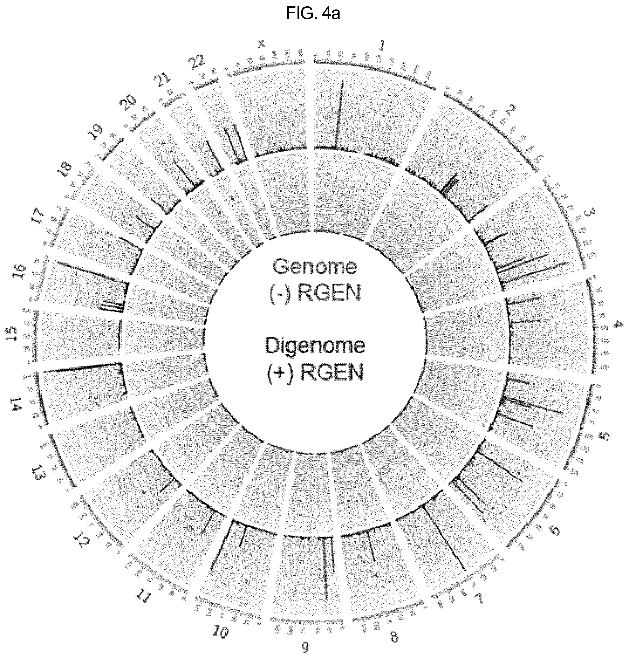

FIG. 4a is a genome-wide Circos plot showing in vitro cleavage sites. Human genomic DNA is shown in red, and RGEN-digested genomic DNA is shown in blue.

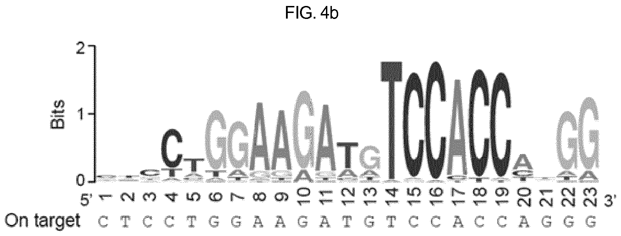

Sequence logos obtained using 42 sequences including 41 Digenome-capture sites and one On-target sequence are given in FIG. 4b.

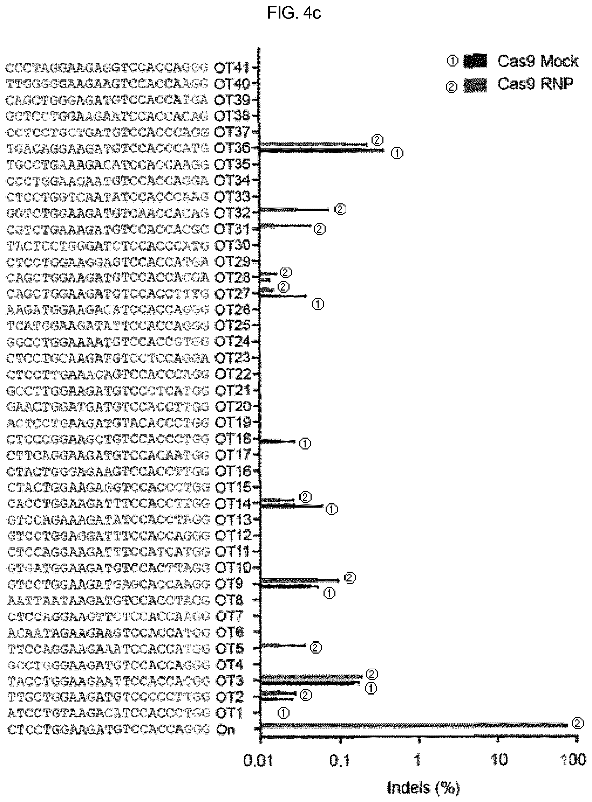

Off-target sites and indel frequencies validated in human ARPE-19 cells by targeted deep sequencing are shown in FIG. 4c. Mismatched nucleotides are shown in red, and PAM sequences are shown in blue.

The particular target sequence of the Vegfa-specific Cas9 RNP shown in FIGS. 4a and 4b is well conserved in the human *VEGFA* gene.

The human genome-wide specificity was assessed using Digenome-seq (Kim et al., Nature Methods 12, 237-243 (2015)) in which cell-free human genomic DNA was treated in vitro using the Vegfa-specific Cas9 RNP and then subjected to whole-genome sequencing. Uniform, rather than random, alignments of sequence reads at in vitro cleavage sites are computationally identified to provide a list of potential off-target sites. Digenome-seq using the Vegfa-specific Cas9 RNP revealed 42 in vitro cleavage sites including the on-target site and the sites are summarized in Table 8, below.

**Table 8**

| *In vitro* cleavage sites in the human genome identified by Digenome-seq using the VEGFA sgRNA | | | | | | |
|---|---|---|---|---|---|---|
| **No.** | **Gene** | | **Sequence** | **Chromosome** | **Position** | **Direction** |
| On | *VEGFA* | Exon | CTCCTGGAAGATGTCCACCA GGG (SEQ ID NO: 293) | chr6 | 43745263 | - |
| OT1 | *NAALAD L2* | Intro n | ATCCTGTAAGACATCCACCC TGG (SEQ ID NO: 317) | chr3 | 1746058 31 | - |
| OT2 | Intergenic region | - | TTGCTGGAAGATGTCCCCCT TGG (SEQ ID NO: 318) | chr12 | 2814792 9 | - |
| OT3 | *CPNE4* | Intron | TACCTGGAAGAATTCCACCA CGG (SEQ ID NO: 319) | chr3 | 1314851 98 | - |
| OT4 | *ABTB2* | Intron | GCCTGGGAAGATGTCCACC AGGG (SEQ ID NO: 320) | chr11 | 34241888 | - |
| OT5 | *ARFGEF3* | Intron | [?] TTCCAGGAAGAAATCCACCA TGG (SEQ ID NO: 321) | chr6 | 138531951 | - |
| OT6 | *Intergenic region* | - | ACAATAGAAGAAGTCCACC ATGG (SEQ ID NO: 322) | chr5 | 21242334 | + |
| OT7 | *BAIAP2-AS1* | Exon | CTCCAGGAAGTTCTCCACCA AGG (SEQ ID NO: 323) | chr17 | 79006487 | - |
| OT8 | *HOMER1* | Intron | AATTAATAAGATGTCCACCT ACG (SEQ ID NO: 324) | chr5 | 78719511 | - |
| OT9 | *ABCA3* | Exon | GTCCTGGAAGATGAGCACC AAGG (SEQ ID NO: 325) | chr16 | 2327650 | - |
| OT10 | Intergenic region | - | GTGATGGAAGATGTCCACTT AGG (SEQ ID NO: 326) | chr2 | 1292980 46 | - |
| OT11 | *Intergenic region* | - | [?] CTCCAGGAAGATTTCCATCA TGG (SEQ ID NO: 327) | chr4 | 16957810 | + |
| OT12 | Intergenic region | - | GTCCTGGAGGATTTCCACCA GGG (SEQ ID NO: 328) | chr8 | 70150186 | - |
| OT13 | *DSCAM* | Intron | GTCCAGAAAGATATCCACCT AGG (SEQ ID NO: 329) | chr21 | 42029091 | - |
| OT1 4 | *CLIP2* | Intro n | CACCTGGAAGATTTCCACCT TGG (SEQ ID NO: 330) | chr7 | 7377060 0 | + |
| OT1 5 | Intergenic region | - | CTACTGGAAGAGGTCCACC CTGG (SEQ ID NO: 331) | chr14 | 1037487 91 | + |
| OT1 6 | *ACSS2* | Intro n | CTACTGGGAGAAGTCCACC TTGG (SEQ ID NO: 332) | chr20 | 3350633 0 | + |
| OT1 7 | Intergenic region | - | CTTCAGGAAGATGTCCACAA TGG (SEQ ID NO: 333) | chr4 | 74527941 | + |
| OT1 8 | Intergenic region | - | CTCCCGGAAGCTGTCCACC CTGG (SEQ ID NO: 334) | chr14 | 106089679 | + |
| OT1 9 | *ITIH4, RP5-966M1.6* | Intron | ACTCCTGAAGATGTACACCC TGG (SEQ ID NO: 335) | chr3 | 52863033 | + |
| OT2 0 | Intergenic region | - | GAACTGGATGATGTCCACCT TGG (SEQ ID NO: 336) | chr6 | 1570612 70 | + |
| OT2 1 | *LINC011* 70 | Exon | GCCTTGGAAGATGTCCCTCA TGG (SEQ ID NO: 337) | chr5 | 1236500 52 | + |
| OT2 2 | *Intergenic region* | - | CTCCTTGAAAGAGTCCACCC AGG (SEQ ID NO: 338) | chr2 | 2361086 83 | + |
| OT23 | Intergenic region | - | CTCCTGCAAGATGTCCTCCA GGA (SEQ ID NO: 339) | chr2 | 1204209 42 | - |
| OT24 | Intergenic region | - | GGCCTGGAAAATGTCCACC GTGG (SEQ ID NO: 340) | chr2 | 1229050 21 | + |
| OT25 | *MGAT5* | Intron | TCATGGAAGATATTCCACCA GGG (SEQ ID NO: 341) | chr2 | 1349523 86 | - |
| OT2 6 | *U91319.1* | Intron | AAGATGGAAGACATCCACC AGGG (SEQ ID NO: 342) | chr16 | 1359269 5 | + |
| OT2 7 | Intergenic region | - | CAGCTGGAAGATGTCCACCT TTG (SEQ ID NO: 343) | chr16 | 8425207 9 | + |
| OT2 8 | Intergenic region | - | CAGCTGGAAGATGTCCACC ACGA (SEQ ID NO: 344) | chr1 | 5903790 9 | + |
| OT2 9 | Intergenic region | - | CTCCTGGAAGGAGTCCACC ATGA (SEQ ID NO: 345) | chr5 | 7246379 3 | + |
| OT3 0 | *SLC9A9* | Intron | TACTCCTGGGATCTCCACCC ATG (SEQ ID NO: 346) | chr3 | 1431669 35 | - |
| OT3 1 | *PTPRS* | Exon | CGTCTGAAAGATGTCCACCA CGC (SEQ ID NO: 347) | chr19 | 5207995 | + |
| OT3 2 | Intergenic region | - | GGTCTGGAAGATGTCAACCA CAG (SEQ ID NO: 348) | chr10 | 1311772 01 | - |
| OT3 3 | Intergenic region | - | CTCCTGGTCAATATCCACCC AAG (SEQ ID NO: 349) | chr22 | 2594492 1 | + |
| OT3 4 | *ERC2* | Intron | CCCTGGAAGAATGTCCACC AGGA (SEQ ID NO: 350) | chr3 | 5556357 7 | + |
| OT3 5 | *CTD-2130013* .*1* | Intron | TGCCTGAAAGACATCCACCA AGG (SEQ ID NO: 351) | chr18 | 4483005 3 | - |
| OT3 6 | Intergenic region | - | TGACAGGAAGATGTCCACC CATG (SEQ ID NO: 352) | chr9 | 2897930 8 | + |
| OT3 7 | Intergenic region | - | CCTCCTGCTGATGTCCACCC AGG (SEQ ID NO: 353) | chr16 | 1065437 | + |
| OT3 8 | Intergenic region | - | GCTCCTGGAAGAATCCACC ACAG (SEQ ID NO: 354) | chr10 | 1307538 19 | + |
| OT39 | *BRD1* | Intron | CAGCTGGGAGATGTCCACC ATGA (SEQ ID NO: 355) | chr22 | 5017442 6 | + |
| OT40 | *CACNG3* | Intron | TTGGGGGAAGAAGTCCACC AAGG (SEQ ID NO: 356) | chr16 | 2431023 7 | + |
| OT41 | *RP11-57C13.6, RP11-57C13.3* | Intron | CCCTAGGAAGAGGTCCACC AGGG (SEQ ID NO: 357) | chr10 | 8940473 5 | - |

To examine the validity of the sites listed in Table 8, targeted deep sequencing was performed using genomic DNA isolated from Vegfa-specific RNP-transfected ARPE-19 cells (see FIG. 4c). Although these sites were cleaved efficiently in vitro, off-target indels were not detected at these 41 cleavage sites above sequencing error rates (average 0.1 %).

Modified gRNAs with improved specificity (Fu, Y., Sander, J. D., Reyon, D., Cascio, V. M. & Joung, J. K. Improving CRISPR-Cas nuclease specificity using truncated guide RNAs. Nature biotechnology 32, 279-284 (2014); Cho, S. W. et al. Analysis of off-target effects of CRISPR/Cas-derived RNA-guided endonucleases and nickases. Genome research 24, 132-141 (2014)) or Cas9 variants (Kleinstiver, B. P. et al. High-fidelity CRISPR-Cas9 nucleases with no detectable genome-wide off-target effects. Nature 529, 490-495 (2016); Slaymaker, I. M. et al. Rationally engineered Cas9 nucleases with improved specificity. Science 351, 84-88 (2016)) may be used, if necessary, to further reduce or avoid the off-target effect that exists although slight.

Taken together, these results show that the Vegfa-specific RNP containing the sgRNA that has the Vegfa targeting sequence provided in the present disclosure is highly specific in both mouse and human cells (in vivo and in vitro).

### EXAMPLE 5: Assay for Side-Effect of Vegfa-Specific Cas9 RNP

Another major concern for mutating the Vegfa gene for the treatment of AMD or diabetic retinopathy is the trophic role of Vegfa in the eye. Cone dysfunction is the most significant change upon Vegfa mutating, and is observed 3 days after conditional deletion of the Vegfa gene in mouse RPE.

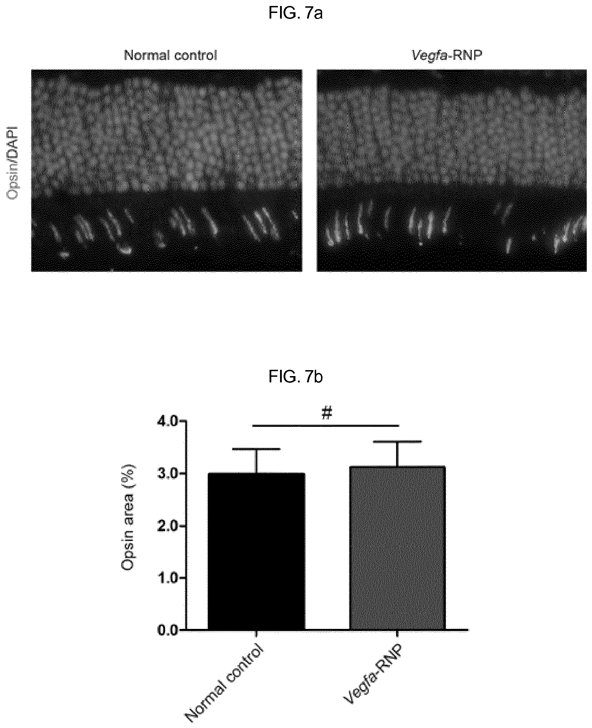

To examine whether the Vegfa-specific Cas9 RNP provided by the present disclosure caused cone dysfunction, opsin-positive areas were observed using a fluorescence microscope and calculated. The results are depicted in FIGS. 7a (fluorescent image) and 7b (opsin positive area (%)).

FIG. 7a shows fluorescent cross-sectional images of the respective retinas obtained 7 days after injection of the Vegfa-specific Cas9 RNP from a normal mouse injected with the *Vegfa-specific Cas9 RNP* (only RNP injection without laser treatment) and a normal control mouse without injection of the Vegfa-specific Cas9 RNP. Opsin appears in green and DAPI is shown in blue. FIG. 7b is a graph showing opsin positive areas (%), with no significant difference between the normal control (3.0±0.5%) and the Vegfa-specific Cas9 RNP-injected mice (3.1±0.5%) (Error bars indicate s.e.m. (n=4); #, *P >* 0.05 (Student's t-test)).

As understood from FIGS. 7a and 7b, the mice injected with the Vegfa-specific Cas9 RNP provided by the present disclosure did not differ in the option positive area of cone cells from the non-injected normal control, even at day 7 after treatment, suggesting that no cone dysfunction had occurred. These results imply that the *Vegfa-*specific Cas9 RNP specifically mutates only target sequences of the *Vegfa* genes, without incurring significant side effects. In addition, the use of Vegfa-specific Cas9 RNP did not generate side effects even 7 days after treatment whereas side effects were observed 3 days after treatment in conventional Vegfa-target therapies.

Herein, some exemplary embodiments of the present invention have been described herein. However, it should be understood by those skilled in the art that these embodiment are provided for illustrative purpose only and should not be construed in any way as limiting the present invention.

## Claims

1. A pharmaceutical composition for use in preventing or treating an eye disease associated with VEGF-A overexpression, wherein the composition comprises a ribonucleoprotein comprising:
a Cas9 protein, and
a VEGF-A gene-specific guide RNA containing a targeting sequence binding specifically to a target site of a VEGF-A gene;
wherein the guide RNA is complementary to a nucleic acid sequence of SEQ ID NO: 293 or 294 in a VEGF-A gene, and
wherein the eye disease associated with VEGF-A overexpression is macular degeneration or retinopathy.

2. The pharmaceutical composition for the use according to claim 1, wherein the guide RNA is complementary to 17-23 bp nucleic acid sequence adjacent to the 5' or 3' end of a PAM sequence in exon 3 or exon 4 of VEGF-A gene.

3. The pharmaceutical composition for the use according to claim 1 or 2, wherein the guide RNA comprises a nucleic acid sequence selected from the group consisting of SEQ ID NOS: 9 to 10.

4. A ribonucleoprotein, comprising a Cas9 protein, and a VEGF-A gene-specific guide RNA comprising a targeting sequence specifically binding to a target site of a VEGF-A gene, wherein the guide RNA is complementary to a nucleic acid sequence of SEQ ID NO: 293 or 294 in a VEGF-A gene.

5. The ribonucleoprotein of claim 4, wherein the guide RNA comprises a nucleic acid sequence selected from the group consisting of SEQ ID NOS: 9 to 10.

## Patentansprüche

1. Pharmazeutische Zusammensetzung zur Verwendung bei der Vorbeugung oder Behandlung einer mit VEGF-A-Überexpression assoziierten Augenerkrankung, wobei die Zusammensetzung ein Ribonukleoprotein umfasst, das Folgendes enthält:
ein Cas9-Protein und
eine VEGF-A-genspezifische guide-RNA, die eine Zielsequenz enthält, die sich spezifisch an eine Zielstelle eines VEGF-A-Gens bindet;
wobei die guide-RNA komplementär zu einer Nukleinsäuresequenz von SEQ ID NO: 293 oder 294 in einem VEGF-A-Gen ist, und
wobei die mit VEGF-A-Überexpression assoziierte Augenerkrankung eine Makuladegeneration oder Retinopathie ist.

2. Pharmazeutische Zusammensetzung zur Verwendung gemäß Anspruch 1, wobei die guide-RNA komplementär zu einer 17-23 bp langen Nukleinsäuresequenz neben dem 5'- oder 3'-Ende einer PAM-Sequenz im Exon 3 oder Exon 4 des VEGF-A-Gens ist.

3. Pharmazeutische Zusammensetzung zur Verwendung gemäß Anspruch 1 oder 2, wobei die guide-RNA eine Nukleinsäuresequenz ausgewählt aus der Gruppe bestehend aus SEQ ID NOS: 9 bis 10 umfasst.

4. Ribonukleoprotein, das ein Cas9-Protein umfasst, und VEGF-A-genspezifische guide-RNA, die eine Zielsequenz umfasst, die sich spezifisch an eine Zielstelle eines VEGF-A-Gens bindet,
wobei die guide-RNA komplementär zu einer Nukleinsäuresequenz von SEQ ID NO: 293 oder 294 in einem VEGF-A-Gen ist.

5. Ribonukleoprotein nach Anspruch 4, wobei die guide-RNA eine Nukleinsäuresequenz ausgewählt aus der Gruppe bestehend aus SEQ ID NOS: 9 bis 10 umfasst.

## Revendications

1. Composition pharmaceutique pour utilisation dans la prévention ou le traitement d'une maladie des yeux associée à la surexpression de VEGF-A, où la composition comprend une ribonucléoprotéine comprenant :
une protéine Cas9, et
un ARN guide spécifique à un gène VEGF-A contenant une séquence de ciblage se liant spécifiquement à un site cible d'un gène VEGF-A ;
dans laquelle l'ARN guide est complémentaire à une séquence d'acide nucléique de la SEQ ID NO : 293 ou 294 dans un gène VEGF-A, et
où la maladie des yeux associée à la surexpression de VEGF-A est une dégénérescence maculaire ou une rétinopathie.

2. Composition pharmaceutique pour utilisation selon la revendication 1, dans laquelle l'ARN guide est complémentaire à une séquence d'acide nucléique de 17-23 paires de base adjacente aux extrémités en 5' ou en 3' d'une séquence PAM dans l'exon 3 ou l'exon 4 du gène VEGF-A.

3. Composition pharmaceutique pour utilisation selon la revendication 1 ou 2, dans laquelle l'ARN guide comprend une séquence d'acide nucléique sélectionnée parmi le groupe composé des SEQ ID NO : 9 à 10.

4. Ribonucléoprotéine, comprenant une protéine Cas9, et un ARN guide spécifique à un gène VEGF-A comprenant une séquence de ciblage se liant spécifiquement à un site cible d'un gène VEGF-A,
dans laquelle l'ARN guide est complémentaire à une séquence d'acide nucléique de la SEQ ID NO : 293 ou 294 d'un gène VEGF-A.

5. Ribonucléoprotéine selon la revendication 4, où l'ARN guide comprend une séquence d'acide nucléique sélectionnée parmi le groupe composé des SEQ ID NO : 9 à 10.
